# EUROPEAN PATENT APPLICATION

(11) **EP 2 520 651 A2**
(43) Date of publication of application: **07.11.2012**
(21) Application number: 10841247.9
(22) Date of filing: 28.12.2010
(51) Int. Cl.: C12N 15/11, C12N 15/63, A61K 31/7105, A61P 35/00

(54) **SIRNA FOR INHIBITING C-MET EXPRESSION AND AN ANTI-CANCER COMPOSITION COMPRISING THE SAME**

(30) Priority: 31.12.2009 KR 20090135665
(71) Applicant: Samyang Biopharmaceuticals Corporation, Seoul 110-725 (KR)
(72) Inventor: KIM, Sun-Ok, Daejeon 305-762 (KR); KIM, Sang-Hee, Daejeon 305-348 (KR); CHO, Eun-ah, Daejeon 305-348 (KR); IN, Chang-hoon, Cheongju-si Chungbuk 360-779 (KR)
(74) Representative: Krauns, Christian
(86) International application number: PCT/KR2010/009440
(87) International publication number: WO 2011/081415

(57) **Abstract**

Disclosed are small interfering RNA (siRNA) that complementarily binds to a base sequence of c-Met transcript (mRNA transcript), thereby inhibiting expression of c-Met without inducing immune responses, and use of the siRNA for prevention and/or treatment of cancer. The siRNA that complementarily binds to c-Met-encoding mRNA may inhibit expression of c-Met, which is commonly overexpressed in almost all cancer cells, through RNA interference (RNAi), thereby inhibiting proliferation and metastasis of cancer cells, and thus, the siRNA may be useful as an anticancer agent.

## Description

### BACKGROUND OF THE INVENTION

### (a) Field of the Invention

The present invention relates to a small interfering RNA (siRNA) that complementary binds to a base sequence of c-Met transcript (mRNA transcript), thereby inhibiting expression of c-Met without eliciting immune responses, and use of the siRNA for prevention and/or treatment of cancer.

### (b) Description of the Related Art

c-Met is a proto-oncogene that encodes a protein known as hepatocyte growth factor receptor (HGFR). Since it has been discovered for the first time in osteosarcoma of human treated with chemical carcinogen at the year of 1984, it was found to be potential proto-oncogene due to genetic fusion with tpr(translocated promoter region) at the year of 1986 (Cooper et al., Nature, 311, 29-33, 1984; Dean et al., Mol cell Biol., 7, 921-924, 1987 ; Park et al., Cell, 45, 895-904, 1986).

On binding to the cell surface receptor tyrosine kinase (TK) known as c-Met, hepatocyte growth factor (HGF) to be secreted by mesenchymal cells stimulates cell growth, cell motility, embryogenesis, wound healing and angiogenesis. These pleiotropic actions are fundamentally important during development, homeostasis, and tissue regeneration. HGF signaling also contributes to oncogenesis and tumor progression in several human cancers and promotes aggressive cellular invasiveness that is strongly linked to tumor metastasis. (Nakamura et al., J Clin Invest., 106, 1511-9,2000; Comoglio et al., Semin Cancer Biol, 11, 153-65, 2001; Boccaccio, Nat Rev Cancer, 6, 637-45, 2006; Huh CF et al., Proc Natl Acad Sci USA, 101, 4477-82, 2004).

Most of abnormal signal transduction of HGF/c-Met results from increase in the activity of HGF or c-Met due to overexpression or mutation thereof, and it is known to be closely related to a bad prognosis in various cancer patients.

Owing to the discovery of close relationship between the activity of c-Met protein and cancer incidence/metastasis and clinical success of other receptor tyrosine kinase inhibitors, development of anticancer drug targeting c-Met is actively progressed. However, currently, most of drug candidates in the clinical step are chemical synthesis inhibitors, such as tyrosine kinase inhibitor that inhibits c-Met signal pathway, to be designed based on the tertiary structure of proteins or antibody to c-Met receptor.

Although low molecular kinase inhibitor has improved selectivity to c-Met compared to kinase inhibitors targeting a large panel of protein kinases, there is still a concern for side effect due to off-targeting other protein which is structurally similar with c-Met protein.

Although antibody drugs comparatively have excellent selectivity, there are problems in terms of productivity by the complicated production process and instability during storage. Therefore, development of effective inhibitor targeting c-Met function is continuously demanded.

Recently, it has been revealed that the ribonucleic acid-mediated interference (RNAi) contributes to development of drug lead-candidate by exhibiting sequence specific gene silencing even for otherwise non-druggable targets with the existing technologies. Therefore, RNAi has been considered as a technology capable of suggesting solutions to the problems of limited targets and non-specificity in synthetic drugs, and overcoming limitations of chemical synthetic drugs, and thus, a lot of studies on the use thereof in development of medicines for various diseases that is hard to be treated by the existing technologies, in particular cancer, are actively progressed.

However, it was found out that siRNA(small interfering RNA) triggers innate immune responses, and also induces non-specific RNAi effect more frequently than expected.

It has been reported that in mammal cells, long double stranded siRNA may induce a deleterious interferon response; short double stranded siRNA may also induce an initial interferon response deleterious to the human body or cells; and many siRNAs have been known to induce higher non-specific RNAi effect than expected (Kleirman et al. Nature, 452:591-7, 2008).

Although there has been an attempt to develop siRNA anticancer drugs targeting c-Met which plays an important role in the progression of cancer, so far the outcome is insignificant. Gene inhibition effect of individual sequence of siRNA has not been suggested, and particularly, immune activity has not been considered.

Although siRNA shows great promise as a novel medicine due to the advantages such as high activity, excellent target specificity, and the like, it has several obstacles to overcome for therapeutic development, such as low blood stability because it may be degraded by nuclease in blood, a poor ability to pass through cell membrane due to negative charge, short half life in blood due to rapid excretion, whereby its limited tissue distribution, and induction of off-target effect capable of affecting on regulation pathway of other genes.

Recently, in order to improve these disadvantages and enable the application to clinical test, studies are progressed on introducing chemical modification in siRNA (Davidson, Nat. Biotechnol., 24:951-952,2006; Sioud and Furset, J. Biomed. Biotechnol., 2006:23429,2006).

### SUMMARY OF THE INVENTION

Accordingly, the inventors developed siRNA that has high sequence specificity and thus specifically binds to transcript of a target gene to increase RNAi activity, and does not induce any immune toxicity, and completed the invention.

One embodiment provides siRNA that complementarily binds to c-Met mRNA transcript, thereby specifically inhibiting synthesis and/or expression of c-Met.

Another embodiment provides an expression vector for expressing the siRNA.

Another embodiment provides a pharmaceutical composition for inhibiting synthesis and/or expression of c-Met, comprising the siRNA or the siRNA expression vector as an active ingredient.

Another embodiment provides an anticancer composition comprising the siRNA or the siRNA expression vector as an active ingredient.

Another embodiment provides a method for inhibiting synthesis and/or expression of c-Met comprising preparing the above siRNA or the siRNA expression vector; and contacting the siRNA or the siRNA expression vector with c-Met-expressing cells, and use of the siRNA or the siRNA expression vector for inhibition of synthesis and/or expression of c-Met in c-Met-expressing cells.

Yet another embodiment provides a method for inhibiting growth of cancer cells comprising preparing the above siRNA or the siRNA expression vector; and contacting the siRNA or the siRNA expression vector with c-Met-expressing cancer cells, and use of the siRNA or the siRNA expression vector for inhibiting growth of cancer cells in c-Met-expressing cancer cells.

Yet another embodiment provides a method for preventing and/or treating cancer comprising preparing the siRNA or the siRNA expression vector; and administering the siRNA or the siRNA expression vector to a patient in a therapeutically effective amount, and use of the siRNA or the siRNA expression vector for prevention and/or treatment of cancer.

### BRIEF DESCRIPTION OF THE DRAWINGS

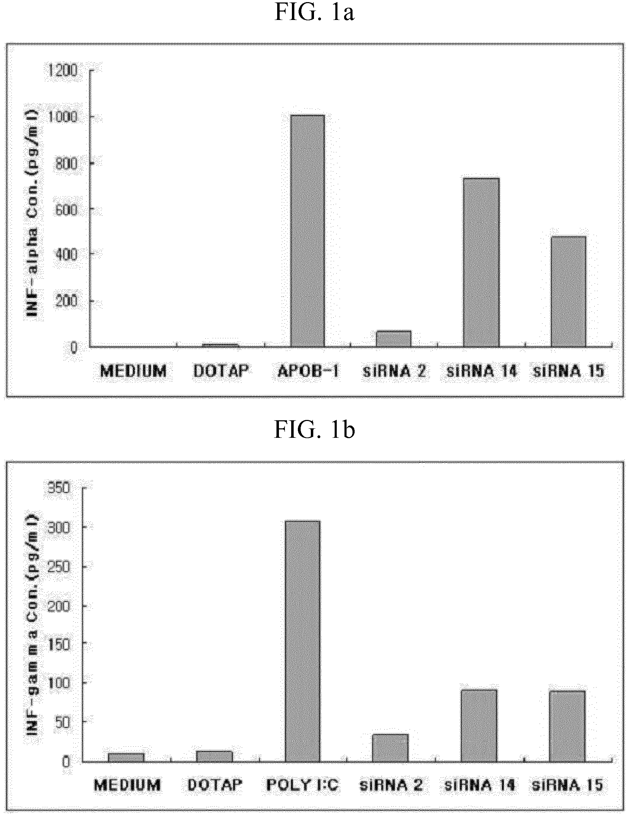

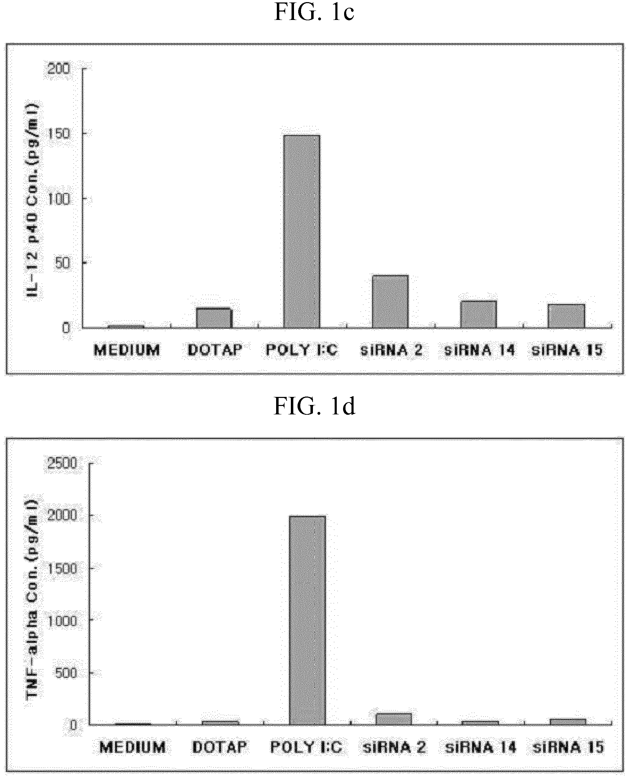

Fig. 1a to Fig. 1d shows change in cytokine concentration according to siRNA treatment, wherein 1 a denotes the concentration of interferon alpha, 1b denotes the concentration of interferon gamma, 1 c denotes the concentration of interleukin-12, and 1 d denotes the concentration of tumor necrosis factor.

### DETAILED DESCRIPTION OF THE EMBODIMENTS

The present invention provides siRNA that complementarily binds to c-Met mRNA transcript base sequence, thereby inhibiting synthesis and/or expression of c-Met in the cells, a pharmaceutical composition comprising the same, and use thereof.

According to one aspect of the present invention, provided is siRNA for specifically inhibiting synthesis and/or expression of c-Met. According to another aspect, provided is a pharmaceutical composition for inhibiting synthesis and/or expression of c-Met, comprising the siRNA specifically inhibiting synthesis and/or expression of c-Met as an active ingredient. According to yet another aspect, provided is an agent for inhibiting cancer cell growth, or a pharmaceutical composition (anticancer composition) for prevention and/or treatment of a cancer, comprising the siRNA specifically inhibiting synthesis and/or expression of c-Met as an active ingredient.

The present invention relates to a technology of inhibiting expression of c-Met mRNA in mammals including human, an alternative splice form or a mutant thereof, or c-Met gene of the same lineage, which may be achieved by administering a specific amount of the siRNA of the present invention to a patient, to reduce the target mRNA.

Hereinafter, the present invention will be described in detail.

The c-Met may be originated from mammals, preferably human or it may be c-Met of the same lineage as human and a mutant thereof. The term 'same lineage as human' refers to mammals having genes or mRNA of 80% or more sequence homology with human c-Met genes or mRNA originated therefrom, and specifically, it may include human, primates, rodents, and the like.

According to one embodiment, cDNA sequence of a sense strand corresponding to c-Met-encoding mRNA may be SEQ ID NO 1.

The siRNA according to the present invention may target mRNA or cDNA region corresponding to at least one base sequence selected from the group consisting of a region consisting of consecutive 15 to 25 bp, preferably consecutive 18 to 22 bp, preferably consecutive 2 to 21 bp in the mRNA or cDNA of c-Met. Preferable target regions on cDNA are summarized in the following Table 1. Thus, according to one embodiment of the invention, provided is siRNA for targeting the mRNA or cDNA region corresponding to at least one base sequence selected from the group consisting of SEQ ID NOs 2 to 21 in the c-Met cDNA region of SEQ ID NO: 1. Specifically, provided is siRNA for targeting the mRNA region corresponding to base sequence selected from the group consisting of SEQ ID NOs: 3, 18, and 21.

**[Table 1] Target regions on c-Met cDNA(SEQ ID NO: 1)(20)**

| SEQ ID NO. | Sequence (5' -> 3') | Start nucleotide in c-Met gene |
|---|---|---|
| 2 | GTAAAGAGGCACTAGCAAA | 77 |
| 3 | GCACTAGCAAAGTCCGAGA | 86 |
| 4 | CAGCAAAGCCAATTTATCA | 306 |
| 5 | CTATGATGATCAACTCATT | 375 |
| 6 | CAATCATACTGCTGACATA | 444 |
| 7 | CTCTAGATGCTCAGACTTT | 803 |
| 8 | TCTGGATTGCATTCCTACA | 856 |
| 9 | CTGGATTGCATTCCTACAT | 857 |
| 10 | GCACAAAGCAAGCCAGATT | 1039 |
| 11 | CTGCTTTAATAGGACACTT | 1188 |
| 12 | CAGGTTGTGGTTTCTCGAT | 1390 |
| 13 | CTGGTTATCACTGGGAAGA | 1507 |
| 14 | TTGGTCCTGCCATGAATAA | 1877 |
| 15 | AGACAAGCATCTTCAGTTA | 2195 |
| 16 | TCGCTCTAATTCAGAGATA | 2376 |
| 17 | TCAGAGATAATCTGTTGTA | 2386 |
| 18 | GTGAGAATATACACTTACA | 2645 |
| 19 | GGTGTTGTCTCAATATCAA | 2809 |
| 20 | CATTTGGATAGGCTTGTAA | 2935 |
| 21 | CCAAAGGCATGAAATATCT | 3566 |

As used herein, the term 'target mRNA' refers to human c-Met mRNA, c-Met mRNA of the same lineage as human, a mutant, or an alternative splice form thereof. Specifically, it may include mutants of amino acid or base sequence such as NM_000245, Mus musculus: NM_008591, Macaca mulatta: NM_001168629, NM_001127500: a splice form wherein base sequence 2262∼2317 are deleted, Y1230C/A3689G, D1228H/G3682C, V1092I/G3274A, M1268T/T3795C, and the like. Thus, the siRNA of the present invention may target c-Met mRNA of human or the same lineage as human, an alternative splice form, or a mutant thereof.

As used herein, the wording 'targeting mRNA (or cDNA) region' means that siRNA has a base sequence complementary to the base sequence of the whole or a part of the mRNA (or cDNA) region, for example, complementary to 85∼100% of the whole base sequence, thus capable of specifically binding to the mRNA (or cDNA) region.

As used herein, the term 'complementary' or 'complementarily' means that both strands of polynucleotide may form a base pair. Both strands of complementary polynucleotide forms a Watson-Crick base pair to form double strands. When the base U is referred to herein, it may be substituted by the base T unless otherwise indicated.

Since the inhibition effect on c-Met synthesis and/or expression and cancer therapeutic effect of the pharmaceutical composition of the present invention is achieved by effective inhibition on c-Met synthesis and/or expression, siRNA contained in the pharmaceutical composition as an active ingredient may be double stranded siRNA of 15-30 bp that targets at least one of the specific mRNA regions as described above. According to a preferable embodiment, the siRNA may include at least one selected from the group consisting of SEQ ID NOs 22 to 98. More specifically, the siRNA may be at least one selected from the group consisting of siRNA 1 to siRNA 40 as described in the following Table 2.

**[Table 2]**

| | SEQ ID NO | Sequence (5' -> 3') | Strand | siRNA designation | Chemical structural modification |
|---|---|---|---|---|---|
| Double stranded symmetric | 22 | GUAAAGAGGCACUAGCAAAdTdT | Sense | siRNA 1 | |
| | 23 | UUUGCUAGUGCCUCUUUACdTdT 10 | Antisense | | |
| siRNA (20) | 24 | GCACUAGCAAAGUCCGAGAdTdT | Sense | siRNA 2 | |
| | 25 | UCUCGGACUUUGCUAGUGCdTdT | Antisense | | |
| | 26 | CAGCAAAGCCAAUUUAUCAdTdT | Sense | siRNA 3 | |
| | 27 | UGAUAAAUUGGCUUUGCUGdTdT | Antisense | | |
| | 28 | CUAUGAUGAUCAACUCAUUdTdT | Sense | siRNA 4 | |
| | 29 | AAUGAGUUGAUCAUCAUAGdTdT | Antisense | | |
| | 30 | CAAUCAUACUGCUGACAUAdTdT | Sense | siRNA 5 | |
| | 31 | UAUGUCAGCAGUAUGAUUGdTdT | Antisense | | |
| | 32 | CUCUAGAUGCUCAGACUUUdTdT | Sense | siRNA 6 | |
| | 33 | AAAGUCUGAGCAUCUAGAGdTdT | Antisense | | |
| | 34 | UCUGGAUUGCAUUCCUACAdTdT | Sense | siRNA 7 | |
| | 35 | UGUAGGAAUGCAAUCCAGAdTdT | Antisense | | |
| | 36 | CUGGAUUGCAUUCCUACAUdTdT | Sense | siRNA 8 | |
| | 37 | AUGUAGGAAUGCAAUCCAGdTdT | Antisense | | |
| | 38 | GCACAAAGCAAGCCAGAUUdTdT | Sense | siRNA 9 | |
| | 39 | AAUCUGGCUUGCUUUGUGCdTdT | Antisense | | |
| | 40 | CUGCUUUAAUAGGACACUUdTdT | Sense | siRNA 10 | |
| | 41 | AAGUGUCCUAUUAAAGCAGdTdT | Antisense | | |
| | 42 | CAGGUUGUGGUUUCUCGAUdTdT | Sense | siRNA 11 | |
| | 43 | AUCGAGAAACCACAACCUGdTdT | Antisense | | |
| | 44 | CUGGUUAUCACUGGGAAGAdTdT | Sense | siRNA 12 | |
| | 45 | UCUUCCCAGUGAUAACCAGdTdT | Antisense | | |
| | 46 | UUGGUCCUGCCAUGAAUAAdTdT | Sense | siRNA 13 | |
| | 47 | UUAUUCAUGGCAGGACCAAdTdT | Antisense | | |
| | 48 | AGACAAGCAUCUUCAGUUAdTdT | Sense | siRNA 14 | |
| | 49 | UAACUGAAGAUGCUUGUCUdTdT | Antisense | | |
| | 50 | UCGCUCUAAUUCAGAGAUAdTdT | Sense | siRNA 15 | |
| | 51 | UAUCUCUGAAUUAGAGCGAdTdT | Antisense | | |
| | 52 | UCAGAGAUAAUCUGUUGUAdTdT | Sense | siRNA 16 | |
| | 53 | UACAACAGAUUAUCUCUGAdTdT | Antisense | | |
| | 54 | GUGAGAAUAUACACUUACAdTdT | Sense | siRNA 17 | |
| | 55 | UGUAAGUGUAUAUUCUCACdTdT | Antisense | | |
| | 56 | GGUGUUGUCUCAAUAUCAAdTdT | Sense | siRNA 18 | |
| | 57 | UUGAUAUUGAGACAACACCdTdT | Antisense | | |
| | 58 | CAUUUGGAUAGGCUUGUAAdTdT | Sense | siRNA 19 | |
| | 59 | UUACAAGCCUAUCCAAAUGdTdT | Antisense | | |
| | 60 | CCAAAGGCAUGAAAUAUCUdTdT | Sense | siRNA 20 | |
| | 61 | AGAUAUUUCAUGCCUUUGGdTdT | Antisense | | |
| Double stranded asymmetric siRNA | 62 | CUAGCAAAGUCCGAGA | Sense | siRNA 21 | |
| | 25 | UCUCGGACUUUGCUAGUGCdTdT | Antisense | | |
| | 63 | AGAAUAUACACUUACA | Sense | siRNA 22 | |
| | 55 | UGUAAGUGUAUAUUCUCACdTdT | Antisense | | |
| (3) | 64 | GAUUGCAUUCCUACAU | Sense | siRNA 23 | |
| | 61 | AGAUAUUUCAUGCCUUUGGdTdT | Antisense | | |
| Chemically modified siRNA (17) | 65 | GCACUAGCAAAGUCCGAGAdT*dT | Sense | siRNA24 | siRNA2 -mod1 |
| | 66 | UCUCGGACUUUGCUAGUGCdT*dT | Antisense | | |
| | 67 | GCACUAGCAAAGUCCGAGAdT*dT | Sense | siRNA25 | siRNA2 -mod2 |
| | 68 | UCUCGGACUUUGCUAGUGCdT*dT | Antisense | | |
| | 69 | GCACUAGCAAAGUCCGAGAdT*dT | Sense | siRNA26 | siRNA2 -mod3 |
| | 70 | UCUCGGACUUUGCUAGUGCdT*dT | Antisense | | |
| | 71 | GCACuAGCAAAGuCCGAGAdT*dT | Sense | siRNA27 | siRNA2 -mod4 |
| | 72 | UCuCGGACuUUGCuAGuGCdT*dT | Antisense | | |
| | 73 | GCACUAGCAAAGUCCGAGAdT*dT | Sense | siRNA28 | siRNA2 -mod5 |
| | 74 | UCUCGGACUUUGCUAGUGCdT*dT | Antisense | | |
| | 75 | GUGAGAAUAUACACUUACAdT*dT | Sense | siRNA29 | siRNA17 -mod1 |
| | 76 | UGUAAGUGUAUAUUCUCACdT*dT | Antisense | | |
| | 77 | GUGAGAAUAUACACUUACAdT*dT | Sense | siRNA30 | siRNA17 -mod2 |
| | 78 | UGUAAGUGUAUAUUCUCACdT*dT | Antisense | | |
| | 79 | GUGAGAAUAUACACUUACAdT*dT | Sense | siRNA31 | siRNA17 -mod3 |
| | 80 | UGUAAGUGUAUAUUCUCACdT*dT | Antisense | | |
| | 81 | GuGAGAAuAuACACuuACAdT*dT | Sense | siRNA32 | siRNA17 |
| | 82 | UGuAAGuGuAUAuuCuCACdT*dT | Antisense | | -mod4 |
| | 83 | GUGAGAAUAUACACUUACAdT*dT | Sense | siRNA33 | siRNA17 |
| | 84 | UGUAAGUGUAUAUUCUCACdT*dT | Antisense | | -mod5 |
| | 85 | GUGAGAAUAUACACUUACAdT*dT | Sense | siRNA34 | siRNA17 |
| | 86 | UGUAAGUGUAUAUUCUCACdT*dT | Antisense | | -mod6 |
| | 87 | CCAAAGGCAUGAAAUAUCUdT*dT | Sense | siRNA35 | siRNA20 |
| | 88 | AGAUAUUUCAUGCCUUUGGdT*dT | Antisense | | -mod1 |
| | 89 | CCAAAGGCAUGAAAUAUCUdT*dT | Sense | siRNA36 | siRNA20 |
| | 90 | AGAUAUUUCAUGCCUUUGGdT*dT | Antisense | | -mod2 |
| | 91 | CCAAAGGCAUGAAAUAUCUdT*dT | Sense | siRNA37 | siRNA20 |
| | 92 | AGAUAUUUCAUGCCUUUGGdT*dT | Antisense | | -mod3 |
| | 93 | CCAAAGGCAuGAAAuAuCudT*dT | Sense | siRNA38 | siRNA20 |
| | 94 | AGAuAuuuCAUGCCuuuGGdT*dT | Antisense | | -mod4 |
| | 95 | CCAAAGGCAUGAAAUAUCUdT*dT | Sense | siRNA39 | siRNA20 |
| | 96 | AGAUAUUUCAUGCCUUUGGdT*dT | Antisense | | -mod5 |
| | 97 | CCAAAGGCAUGAAAUAUCUdT*dT | Sense | siRNA40 | siRNA20 |
| | 98 | AGAUAUUUCAUGCCUUUGGdT*dT | Antisense | | -mod6 |

The notation and contents of the chemical structural modification of chemically modified siRNA (SEQ ID NOs 65 to 98) in the Table 2 are described in the following Table 3 and Table 4.

**[Table 3]**

| Notation | Introduced chemical modification |
|---|---|
| * | Substitution of a phosphodiester linkage with a phosphorothioate linkage |
| underline | Substitution of 2'-OH of the ribose ring with 2'-O-Me |
| Lower case letter | Substitution of 2'-OH of the ribose ring with 2'-F |
| Bold letter | Introduction of ENA(ethylene bridge nucleic acid) |

**[Table 4]**

| Structure name | siRNA chemical modification |
|---|---|
| mod1 | 2'-OH of ribose of 1 ^{st}and 2^{nd} nucleic acids of antisense strand are substituted with 2'-O-Me, and 3' end dTdT (phosphodiester linkage) of sense and antisense strands are substituted with a phosphorothioate linkage (3'-dT*dT, *:phosphorothioate linkage) |
| mod2 | in addition to mod1 modification, 2'-OH groups of riboses of 1st and 2nd nucleic acids of sense strand are substituted with 2'-O-Me |
| mod3 | in addition to mod2 modification, 2'-OH groups of riboses of all U containing nucleic acids of sense strand are substituted with 2'-O-Me |
| mod4 | in addition to mod1 modification, 2'-OH groups of riboses of all G containing nucleic acids of sense and antisense strands are substituted with 2'-O-Me, and 2'-OH groups of riboses of all U containing nucleic acids of sense and antisense strands are substituted with 2'-F. Provided that 1 0^{th}, 11^{th} bases of antisense strand are not substituted. |
| mod5 | in addition to mod2 modification, ENA(2'-O, 4'-C ethylene bridged nucleotide) is introduced in one 5' end nucleic acid of sense strand. |
| mod6 | 2'-OH group of ribose of 2^{nd} nucleic acid of antisense strand is substituted with 2'-O-Me, and 3' end dTdT (phosphodiester linkage) of sense and antisense strands are substituted with a phosphorothioate linkage (3'-dT*dT, *:phosphorothioate linkage) |

Since the siRNA has high sequence specificity for a specific target region of c-Met mRNA transcript, it can specifically complementarily bind to the transcript of a target gene, thereby increasing RNA interference activity, thus having excellent activity of inhibiting c-Met expression and/or synthesis in cells. And, the siRNA has minimal immune response inducing activity.

As described above, the siRNA of the present invention may be siRNA targeting at least one region of mRNA selected from the group consisting of SEQ ID NOs. 2 to 21 of the c-Met cDNA region of SEQ ID NO. 1. Preferably, the siRNA may comprise at least one nucleotide sequence selected from the group consisting of SEQ ID NOs. 22 to 98, and more preferably, at least one selected from the group consisting of 40 kinds of siRNAs of SEQ ID NOs. 22 to 98. The siRNA includes ribonucleic acid sequence itself, and a recombinant vector (expression vector) expressing the same. The expression vector may be a viral vector selected from the group consisting of a plasmid or an adeno-associated virus, a retrovirus, a vaccinia virus, an oncolytic adenovirus, and the like.

The pharmaceutical composition of the present invention may comprise the siRNA as an active ingredient and a pharmaceutically acceptable carrier. The pharmaceutically acceptable carrier may include any commonly used carriers, and for example, it may be at least one selected from the group consisting of water, a saline solution, phosphate buffered saline, dextrin, glycerol, ethanol, and the like, but not limited thereto.

The siRNA may be administered to mammals, preferably human, monkey, or rodents (mouse, rat), and particularly, to any mammals, for example human, who has diseases or conditions related to c-Met expression, or requires inhibition of c-Met expression.

To obtain c-Met inhibition effect while minimizing undesirable side effects such as an immune response, and the like, the concentration of the siRNA in the composition or the use or treatment concentration of the siRNA may be 0.001 to 1000nM, preferably 0.01 to 100nM, more preferably 0.1 to 10nM, but not limited thereto.

The siRNA or the pharmaceutical composition containing the same may treat at least one cancer selected from the group consisting of various solid cancers such as lung cancer, liver cancer, colorectal cancer, pancreatic cancer, stomach cancer, breast cancer, ovarian cancer, renal cancer, thyroid cancer, esophageal cancer, prostate cancer, and the like, osteosarcoma, soft tissue sarcoma, glioma, and the like.

Hereinafter, the structure and the designing process of the siRNA, and a pharmaceutical composition containing the same will be described in detail.

The siRNA does not induce or do decrease the expression of c-Met protein by degrading c-Met mRNA by RNAi pathway.

According to one embodiment, siRNA refers to small inhibitory RNA duplexes that induce RNA interference (RNAi) pathway. Specifically, siRNA is RNA duplexes comprising a sense strand and an antisense strand complementary thereto, wherein both strands comprise 15-30bp, specifically 19-25bp or 27bp, more specifically 19-21 bp. The siRNA may comprise a double stranded region and have a structure where a single strand forms a hairpin or a stem-loop structure, or it may be duplexes of two separated strands. The sense strand may have identical sequence to the nucleotide sequence of a target gene mRNA sequence. A duplex forms between the sense strand and the antisense strand complementary thereto by Watson-Crick base pairing. The antisense strand of siRNA is captured in RISC (RNA-Induced Silencing Complex), and the RISC identifies the target mRNA which is complementary to the antisense strand, and then, induces cleavage or translational inhibition of the target mRNA.

According to one embodiment, the double stranded siRNA may have an overhang of 1 to 5 nucleotides at 3' end, 5' end, or both ends. Alternatively, it may have a blunt end truncated at both ends. Specifically, it may be siRNA described in US20020086356, and US7056704, which are incorporated herein by reference.

According to one embodiment, the siRNA comprises a sense strand and an antisense strand, wherein the sense strand and the antisense strand form a duplex of 15-30 bp, and the duplex may have a symmetrical structure having a blunt end without an overhang, or an asymmetric structure having an overhang of at least one nucleotide, for example 1-5 nucleotides. The nucleotides of the overhang may be any sequence, but it may have 2 dTs (deoxythymidine) attached thereto.

The antisense strand is hybridized with the target region of mRNA of SEQ ID NO. 1 under a physiological condition. The description 'hybridized under physiological condition' means that the antisense strand of the siRNA is *in vivo* hybridized with a specific target region of mRNA. Specifically, the antisense strand may have 85% or more sequence complementarity to the target mRNA region, where the target mRNA region is preferably at least one base sequence selected from SEQ ID NOs. 2 to 21 as shown in Table 1, and more specifically, the antisense strand may comprise a sequence completely complementary to consecutive 15 to 25 bp, preferably consecutive 18 to 22 bp within the base sequence of SEQ ID NO. 1. Still more preferably, the antisense strand of the siRNA may comprise a sequence completely complementary to at least one base sequence selected from SEQ ID NOs. 2 to 21, as shown in Table 1.

According to one embodiment, the siRNA may have an asymmetric double stranded structure, wherein one strand is shorter than the other strand. Specifically, in the case of siRNA (small interfering RNA) molecule of double strands consisting of an antisense strand of 19 to 21 nucleotides (nt) and a sense strand of 15 to 19 nt having complementary sequence to the antisense, the siRNA may be an asymmetric siRNA having a blunt end at 5' end of the antisense and a 1-5 nucleotides overhang at 3' end of the antisense. Specifically, it may be siRNA disclosed in WO09/078685.

In the treatment using siRNA, it is required to select an optimum base sequence having highest activity in the base sequence of the targeted gene. Specifically, according to one embodiment, to increase relationship between pre-clinical trials and clinical trial, it is preferable to design c-Met siRNA comprising a conserved sequence between species. And, according to one embodiment, it is preferable to design such that the antisense strand binding to RISC may have high binding ability to RISC. Thus, it may be designed such that there may be difference between thermodynamic stabilities between a sense strand and an antisense strand, thus increasing RISC binding ability of the antisense strand that is a guide strand, while the sense strand does not bind to RISC. Specifically, GC content of the sense strand may not exceed 60%; 3 or more adenine/guanine bases may exist in the 15^{th} to 19^{th} positions from 5' end of the sense strand; and G/C bases may be abundant in the 1^{st} to 7^{th} positions from 5' end of the sense strand. And, since due to repeated base sequences, internal sequences of siRNA itself may bind to each other and lower the ability of complementary binding to mRNA, it may be preferable to design such that less than 4 repeated base sequences exist. And, in the case of a sense strand consisting of 19 bases, to bind to mRNA of a target gene to properly induce degradation of the transcript, 3^{rd}, 10^{th}, and 19^{th} bases from 5' end of the sense strand may be adenine.

Further, according to one embodiment, siRNA has minimized non-specific binding and immune response-inducing activity. The inducing of an immune response of interferon, and the like by siRNA mostly occurs through TLR7 (Toll-like receptor-7) that exists at endosome of antigen-presenting immune cells, and binding of siRNA to TLR7 occurs in a sequence specific manner like in GU rich sequences, and thus, it may be best to comprise a sequence that is not recognized by TLR7. Specifically, it may not have an immune response-inducing sequence such as 5'-GUCCUUCAA-3' and 5'-UGUGU-3', and have 70% or less complementarity to genes other than c-Met.

Examples of the c-Met cDNA target sequence include the nucleotides of the sequences described in the above Table 1. Based on the target sequences of Table 1, siRNA sequence may be designed such that siRNA length may be longer or shorter than the length of the target sequence, or nucleotides complementary to the DNA sequences may be added or deleted.

According to one embodiment of the invention, siRNA may comprise a sense strand and an antisense strand, wherein the sense strand and the antisense strand form double strands of 15-30 bp without an overhang, or at least one end may have an overhang of 1-5 nucleotides, and the antisense strand may be hybridized to the mRNA region corresponding to any one of SEQ ID NOs 2 to 21, preferably SEQ ID NO 3, 18, 21, under physiological condition. Namely, the antisense strand comprises a sequence complementary to any one of SEQ ID NOs 2 to 21, preferably to SEQ ID NOs 3, 18, 21. Thus, the c-Met siRNA and the pharmaceutical composition containing the same of the present invention do not induce a harmful interferon response and yet inhibit expression of c-Met gene.

The present invention inhibits expression of c-Met in cells by complementary binding to the mRNA region corresponding to at least one sequence selected from the group consisting of SEQ ID NO 3 (5'-GCACTAGCAAAGTCCGAGA-3'), SEQ ID NO 18 (5'-GTGAGAATATACACTTACA-3'), and SEQ ID NO 21 (5'-CCAAAGGCATGAAATATCT-3').

The c-Met siRNA according to specific embodiments of the invention are as described in the above Table 2.

According to one embodiment, the c-Met siRNA may be at least one selected from the group consisting of siRNA 2 comprising a sense sequence of SEQ ID NO 24 and an antisense sequence of SEQ ID NO 25, siRNA 17 comprising a sense sequence of SEQ ID NO 54 and an antisense sequence of SEQ ID NO 55, siRNA 20 comprising a sense sequence of SEQ ID NO 60 and an antisense sequence of SEQ ID NO 61, siRNA 21 comprising a sense sequence of SEQ ID NO 62 and an antisense sequence of SEQ ID NO 25, siRNA 22 comprising a sense sequence of SEQ ID NO 63 and an antisense sequence of SEQ ID NO 55, and siRNA 23 comprising a sense sequence of SEQ ID NO 64 and an antisense sequence of SEQ ID NO 61.

Knockdown (c-Met expression inhibition) may be confirmed by measuring change in the mRNA or protein level by quantitative PCR (qPCR) amplification, bDNA (branched DNA) assay, Western blot, ELISA, and the like. According to one embodiment, a liposome complex is prepared to treat cancer cell lines, and then, ribonucleic acid-mediated interference of expression may be confirmed by bDNA assay in mRNA stage.

The siRNA sequence of the present invention has low immune response inducing activity while effectively inhibiting synthesis or expression of c-Met.

According to one embodiment, immune toxicity may be confirmed by treating human peripheral blood mononuclear cells (PBMC) with an siRNA-DOTAP(N-[1-(2,3-Dioleoyloxy)propyl]-N,N,N-trimetylammonium methylsulfate) complex, and then, measuring whether released cytokines of INF-α and INF-γ, tumor necrosis factor-α (TNF-α), interleukin-12 (IL-12), and the like are increased or not in the culture fluid.

The siRNA may have a naturally occurring (unmodified) ribonucleic acid unit structure, or it may be chemically modified, and for example, it may be synthesized such that the sugar or base structure of at least one ribonucleic acid, a linkage between ribonucleic acids may have at least one chemical modification.

Through the chemical modification of siRNA, desirable effects such as improved resistance to nuclease, increased intracellular uptake, increased cell targeting (target specificity), increased stability, or decreased off-target effect such as decreased interferon activity, immune response and sense effect, and the like may be obtained without influencing the original RNAi activity.

The chemical modification method of siRNA is not specifically limited, and one of ordinary skills in the art may synthesize and modify the siRNA as desired by a method known in the art (Andreas Henschel, Frank Buchholz1 and Bianca Habermann (2004) DEQOR: a web based tool for the design and quality control of siRNAs. Nucleic Acids Research 32(Web Server Issue):W113-W120).

For example, a phosphodiester linkage of siRNA sense and antisense strands may be substituted with a boranophosphate or a phosphorothioate linkage to increase resistance to nucleic acid degradation. For example, a 3' end phosphodiester linkage of siRNA sense and antisense strands may be modified with a phosphorothioate linkage.

For another example, ENA(Ethylene bridge nucleic acid) or LNA(Locked nucleic acid) may be introduced at 5' or 3' end, or both ends of siRNA sense or antisense strand, and preferably, it may be introduced at 5' end of siRNA sense strand. Thereby, siRNA stability may be increased, and an immune response and non-specific inhibition may be reduced, without influencing the RNAi activity.

For yet another example, a 2'-OH group of ribose ring may be substituted with -NH₂ (amino group), -C-allyl(allyl group), -F(fluoro group), or -O-Me (or CH₃, methyl group). For example, 2'-OH group of ribose of 1 st and 2nd nucleic acids of sense strand may be substituted with 2'-O-Me, 2'-OH groups of ribose of 2^{nd} nucleic acid of antisense strand may be substituted with 2'-O-Me, or 2'-OH of riboses of guanine (G) or uridine (U) containing nucleotides may be substituted with 2'-O-Me (methyl group) or 2'-F (fluoro group).

In addition to the above described chemical modifications, various chemical modifications may be made, and only one chemical modification may be made or a plurality of chemical modifications may be made in combination.

In the chemical modification, it is preferable that the activity of knockdown of gene expression may not be reduced while stabilizing the double stranded structure of the siRNA, and thus, minimal modification may be preferred.

And, a ligand such as cholesterol, biotin, or cell penetrating peptide may be attached to 5'- or 3'-end of sense strand of siRNA.

The siRNA of the present invention may be manufactured by in vitro transcription or by cleaving long double stranded RNA with dicer or other nuclease having similar activities. Alternatively, as described above, siRNA may be expressed through a plasmid or a viral expression vector, and the like.

A candidate siRNA sequence may be selected by experimentally confirming whether or not a specific siRNA sequence induces interferon in human peripheral blood mononuclear cells (PBMC) comprising dendritic cells, and then, selecting sequences which do not induce an immune response.

Hereinafter, a drug delivery system (DDS) for delivering the siRNA will be described.

A nucleic acid delivery system may be utilized to increase intracellular delivery efficiency of siRNA.

The system for delivering nucleic acid into cells may include a viral vector, a non-viral vector, liposome, cationic polymer, micelle, emulsion, solid lipid nanoparticles, and the like. The non-viral vector may have high delivery rate and long retention time. The viral vector may include a retroviral vector, an adenoviral vector, a vaccinia virus vector, an adeno-associated viral vector, an oncolytic adenovirus vector, and the like. The nonviral vector may include plasmid. In addition, various forms such as liposome, cationic polymer, micelle, emulsion, solid lipid nanoparticles, and the like may be used. The cationic polymer for delivering nucleic acid may include natural polymer such as chitosan, atelocollagen, cationic polypeptide, and the like and synthetic polymer such as poly(L-lysine), linear or branched polyethylene imine (PEI), cyclodextrin-based polycation, dendrimer, and the like.

The siRNA or complex of the siRNA and nucleic acid delivery system (pharmaceutical composition) of the present invention may be in vivo or ex vivo introduced into cells for cancer therapy. As shown by the following Examples, if the siRNA or complex of the siRNA and nucleic acid delivery system of the present invention is introduced into cells, it may selectively decrease the expression of target protein c-Met or modify mutation in the target gene to inhibit expression of c-Met involved in oncogenesis, and thus, cancer cells may be killed and cancer may be treated.

The siRNA or a pharmaceutical composition comprising the same of the present invention may be formulated for topical, oral or parenteral administration, and the like. Specifically, the administration route of siRNA may be topical such as ocular, intravaginal, or intraanus, and the like, parenteral such as intarpulmonary, intrabronchial, intranasal, intraepithelial, intraendothelial, intravenous, intraarterial, subcutaneous, intraabdominal, intramuscular, intracranial (intrathecal or intraventricular), and the like, or oral, and the like. For topical administration, the siRNA or the pharmaceutical composition comprising the same may be formulated in the form of a patch, ointment, lotion, cream, gel, drop, suppository, spray, solution, powder, and the like. For parenteral administration, intrathecal or intraventricular administration, the siRNA or pharmaceutical composition containing the same may comprise a sterilized aqueous solution containing appropriate additives such as buffer, diluents, penetration enhancer, other pharmaceutically acceptable carriers or excipients.

Further, the siRNA may be mixed with an injectable solution and administered by intratumoral injection in the form of an injection, or it may be mixed with a gel or adhesive composition for transdermal delivery and directly spread or adhered to an affected area to be administered by transdermal route. The injectable solution is not specifically limited, but preferably, it may be an isotonic aqueous solution or suspension, and may be sterilized and/or contain additives (for example, antiseptic, stabilizer, wetting agent, emulsifying agent, solubilizing agent, a salt for controlling osmotic pressure, buffer and/or liposomalizing agent). The gel composition may contain a conventional gelling agent such as carboxymethyl cellulose, methyl cellulose, acrylic acid polymer, carbopol, and the like and a pharmaceutically acceptable carrier and/or a liposomalizing agent. And, in the adhesive composition for transdermal delivery, an active ingredient layer may include an adhesive layer, a layer for adsorbing sebum and a drug layer, and the drug layer may contain a pharmaceutically acceptable carrier and/or a liposomalizing agent, but not limited thereto.

Further, the siRNA or pharmaceutical composition comprising the same of the present invention may further comprise anticancer chemotherapeutics in addition to the c-Met siRNA, or it may further comprise siRNA for inhibiting expression of at least one selected from the group consisting of growth factors, growth factor receptor, downstream signal transduction protein, viral oncogene, and anticancer drug resistant gene.

Thus, combination of chemotherapy with c-Met siRNA may increase sensitivity to chemotherapeutics thus maximizing therapeutic effects and decreasing side effects, and combination of siRNA for inhibiting expression of various growth factors (VEGF, EGF, PDGF, and the like), growth factor receptor, downstream signal transduction protein, viral oncogene, and anticancer drug resistant gene with the siRNA for inhibiting the expression of c-Met of the present invention may simultaneously block various cancer pathways to maximize anticancer effects.

The anticancer chemotherapeutics that may be used for combined administration with the siRNA for inhibiting the expression of c-Met of the present invention may include cisplatin, carboplatin, oxaliplatin, doxorubicin, daunorubicin, epirubicin, idarubicin, mitoxantrone, valubicin, curcumin, gefitinib, erlotinib, irinotecan, topotecan, vinblastine, vincristine, docetaxel, paclitaxel, and a combination thereof.

According to another embodiment of the invention, provided is a method for inhibiting expression and/or synthesis of c-Met, comprising preparing the effective amount of the c-Met siRNA for inhibiting expression and/or synthesis of c-Met; and contacting the siRNA with c-Met-expressing cells.

According to yet another embodiment, provided is a method for inhibiting growth of cancer cells, comprising preparing the effective amount of the c-Met siRNA for inhibiting synthesis and/or expression of c-Met; and contacting the siRNA with c-Met-expressing cancer cells.

According to yet another embodiment, provided is a method for preventing and/or treating cancer, comprising preparing the c-Met siRNA; and administering the siRNA to a patient in a therapeutically effective amount.

The method of preventing and/or treating cancer may further comprise identifying a patient in need of prevention and/or treatment of cancer before the administration.

The cancer that may be treated according to the present invention may be at least one selected from the group consisting of most of the solid cancer (lung cancer, liver cancer, colorectal cancer, pancreatic cancer, stomach cancer, breast cancer, ovarian cancer, renal cancer, thyroid cancer, esophageal cancer, prostate cancer), osteosarcoma, soft tissue sarcoma, glioma, and the like.

The patient may include mammals, preferably, human, monkey, rodents (mouse, rat, and the like), and the like, and particularly, it may include any mammals, for example, human having a disease or condition (for example, cancer) related to c-Met expression or requiring inhibition of c-Met expression.

The effective amount of the siRNA according to the present invention refers to the amount required for administration in order to obtain the effect of inhibiting c-Met expression or synthesis or the resulting cancer cell growth inhibition and the effect of cancer therapy. Thus, it may be appropriately controlled depending on various factors including the kind or severity of disease, kind of administered siRNA, kind of dosage form, age, weight, general health state, gender and diet of a patient, administration time, administration route, and treatment period, combined drug such as combined chemotherapeutic agents , and the like. For example, daily dose may be 0.001 mg/kg - 100 mg/kg, which may be administered at a time or several times in divided dose.

The siRNA complementary to the base sequence of c-Met transcript (mRNA) of the preset invention may inhibit the expression of c-Met that is commonly expressed in cancer cells by RNA-mediated interference (RNAi) to kill the cancer cells, and thus, it may exhibit excellent anticancer effect. And, it may minimize the induction of immune responses.

While most of the existing drugs inhibit the function of already expressed proteins, the RNAi technology of the present invention may selectively inhibit the expression of specific disease inducing proteins with high activity, and degrade the mRNA which is a pre-stage of protein synthesis, and thus, cancer growth and metastasis may be inhibited without inducing side-effects, and it is expected to become a more fundamental cancer therapy.

Further, combination of chemotherapy with the c-Met siRNA may increase the sensitivity to chemotherapeutics, to maximize therapeutic activity and reduce side-effects, and combination of siRNA for inhibiting the expression of various growth factor (VEGF, EFG, PDGF, and the like), growth factor receptor and downstream signal transduction protein, viral oncogene, and anticancer agent resistant gene with the c-Met siRNA may simultaneously block various cancer pathways, thus maximizing anticancer effect.

Hereinafter, the present invention will be described referring to the following examples.

However, these examples are only to illustrate the invention, and the scope of the invention is not limited thereto.

### Example 1. Design of target base sequence to which siRNA for inhibiting c-Met expression may bind

Using siRNA design programs of siDesign Center (Dharmacon), BLOCK-iT™ RNAi Designer (Invitrogen), AsiDesigner (KRIBB), siDirect (University of Tokyo) and siRNA Target Finder (Ambion), a target base sequence to which siRNA may bind was derived from the c-Met mRNA sequence (NM_000245).

**[Table 5] Target base sequence**

| SEQ ID NO | Sequence (5' -> 3') |
|---|---|
| 2 | GTAAAGAGGCACTAGCAAA |
| 3 | GCACTAGCAAAGTCCGAGA |
| 4 | CAGCAAAGCCAATTTATCA |
| 5 | CTATGATGATCAACTCATT |
| 6 | CAATCATACTGCTGACATA |
| 7 | CTCTAGATGCTCAGACTTT |
| 8 | TCTGGATTGCATTCCTACA |
| 9 | CTGGATTGCATTCCTACAT |
| 10 | GCACAAAGCAAGCCAGATT |
| 11 | CTGCTTTAATAGGACACTT |
| 12 | CAGGTTGTGGTTTCTCGAT |
| 13 | CTGGTTATCACTGGGAAGA |
| 14 | TTGGTCCTGCCATGAATAA |
| 15 | AGACAAGCATCTTCAGTTA |
| 16 | TCGCTCTAATTCAGAGATA |
| 17 | TCAGAGATAATCTGTTGTA |
| 18 | GTGAGAATATACACTTACA |
| 19 | GGTGTTGTCTCAATATCAA |
| 20 | CATTTGGATAGGCTTGTAA |
| 21 | CCAAAGGCATGAAATATCT |

### Example 2. Manufacture of siRNA for inhibiting c-Met expression

23 kinds of siRNAs that may bind to the target base sequences designed in Example 1 were obtained from ST Pharm Co. Ltd (Korea). The 23 kinds of siRNA are as described in Table 6, wherein 3' ends of both strands comprise dTdT.

**[Table 6] Base sequence of siRNA for inhibiting c-Met expression**

| SEQ ID NO | Sequence (5' -> 3') | Strand | siRNA designation |
|---|---|---|---|
| 22 | GUAAAGAGGCACUAGCAAAdTdT | Sense | siRNA 1 |
| 23 | UUUGCUAGUGCCUCUUUACdTdT | Antisense | |
| 24 | GCACUAGCAAAGUCCGAGAdTdT | Sense | siRNA 2 |
| 25 | UCUCGGACUUUGCUAGUGCdTdT | Antisense | |
| 26 | CAGCAAAGCCAAUUUAUCAdTdT | Sense | siRNA 3 |
| 27 | UGAUAAAUUGGCUUUGCUGdTdT | Antisense | |
| 28 | CUAUGAUGAUCAACUCAUUdTdT | Sense | siRNA 4 |
| 29 | AAUGAGUUGAUCAUCAUAGdTdT | Antisense | |
| 30 | CAAUCAUACUGCUGACAUAdTdT | Sense | siRNA 5 |
| 31 | UAUGUCAGCAGUAUGAUUGdTdT | Antisense | |
| 32 | CUCUAGAUGCUCAGACUUUdTdT | Sense | siRNA 6 |
| 33 | AAAGUCUGAGCAUCUAGAGdTdT | Antisense | |
| 34 | UCUGGAUUGCAUUCCUACAdTdT | Sense | siRNA 7 |
| 35 | UGUAGGAAUGCAAUCCAGAdTdT | Antisense | |
| 36 | CUGGAUUGCAUUCCUACAUdTdT | Sense | siRNA 8 |
| 37 | AUGUAGGAAUGCAAUCCAGdTdT | Antisense | |
| 38 | GCACAAAGCAAGCCAGAUUdTdT | Sense | siRNA 9 |
| 39 | AAUCUGGCUUGCUUUGUGCdTdT | Antisense | |
| 40 | CUGCUUUAAUAGGACACUUdTdT | Sense | siRNA 10 |
| 41 | AAGUGUCCUAUUAAAGCAGdTdT | Antisense | |
| 42 | CAGGUUGUGGUUUCUCGAUdTdT | Sense | siRNA 11 |
| 43 | AUCGAGAAACCACAACCUGdTdT | Antisense | |
| 44 | CUGGUUAUCACUGGGAAGAdTdT | Sense | siRNA 12 |
| 45 | UCUUCCCAGUGAUAACCAGdTdT | Antisense | |
| 46 | UUGGUCCUGCCAUGAAUAAdTdT | Sense | siRNA 13 |
| 47 | UUAUUCAUGGCAGGACCAAdTdT | Antisense | |
| 48 | AGACAAGCAUCUUCAGUUAdTdT | Sense | siRNA 14 |
| 49 | UAACUGAAGAUGCUUGUCUdTdT | Antisense | |
| 50 | UCGCUCUAAUUCAGAGAUAdTdT | Sense | siRNA 15 |
| 51 | UAUCUCUGAAUUAGAGCGAdTdT | Antisense | |
| 52 | UCAGAGAUAAUCUGUUGUAdTdT | Sense | siRNA 16 |
| 53 | UACAACAGAUUAUCUCUGAdTdT | Antisense | |
| 54 | GUGAGAAUAUACACUUACAdTdT | Sense | siRNA 17 |
| 55 | UGUAAGUGUAUAUUCUCACdTdT | Antisense | |
| 56 | GGUGUUGUCUCAAUAUCAAdTdT | Sense | siRNA 18 |
| 57 | UUGAUAUUGAGACAACACCdTdT | Antisense | |
| 58 | CAUUUGGAUAGGCUUGUAAdTdT | Sense | siRNA 19 |
| 59 | UUACAAGCCUAUCCAAAUGdTdT | Antisense | |
| 60 | CCAAAGGCAUGAAAUAUCUdTdT | Sense | siRNA 20 |
| 61 | AGAUAUUUCAUGCCUUUGGdTdT | Antisense | |
| 62 | CUAGCAAAGUCCGAGA | Sense | siRNA 21 |
| 25 | UCUCGGACUUUGCUAGUGCdTdT | Antisense | |
| 63 | AGAAUAUACACUUACA | Sense | siRNA 22 |
| 55 | UGUAAGUGUAUAUUCUCACdTdT | Antisense | |
| 64 | GAUUGCAUUCCUACAU | Sense | siRNA 23 |
| 61 | AGAUAUUUCAUGCCUUUGGdTdT | Antisense | |

### Example 3: c-Met expression inhibition test in cancer cell line using siRNA

Using each siRNA manufactured in Example 2, human lung cancer cell line (A549, ATCC) and human liver cancer cell line (SK-Hep-1, ATCC) were transformed, and c-Met expression was measured in the transformed cancer cell line.

### Example 3-1. Culture of cancer cell line

Human lung cancer cell line (A549) and human liver cancer cell line (SK-Hep-1) obtained from American Type Culture Collection (ATCC) were cultured at 37□, and 5%(v/v) CO₂, using RPMI culture medium (GIBCO/Invitrogen, USA) containing 10%(v/v) fetal bovine serum, penicillin (100units/ml) and streptomycin (100ug/ml).

### Example 3-2. Preparation of a liposomal complex of siRNA for c-Met expression inhibition

25ul of Opti-MEM medium (Gibco) each containing 10 nM of siRNA of the siRNAs 1 to 23 of Example 2 and Opti-MEM medium containing 0.4ul of lipofectamine 2000 (Invitrogen) per well were mixed in the same volume, and reacted at room temperature for 20 minutes to prepare a liposomal complex of siRNA.

### Example 3-3. Inhibition of c-Met mRNA expression in cancer cell line using c-Met targeting siRNA

The lung cancer cell line and liver cancer cell line cultured in Example 3-1 were respectively seeded in a 96 well-plate at 10⁴ cells per well. After 24 hours, the medium was removed, and Opti-MEM medium was added in an amount of 50µl per well. 50µl of the liposomal complex of siRNA prepared in Example 3-2 was added, and cultured in a cell incubator while maintaining at 37□ and 5%(v/v) CO₂ for 24 hours.

To calculate IC₅₀ value, which is a drug concentration for 50% inhibition of c-Met mRNA expression, lung cancer cell line (A549) was treated with each siRNA of the 7 concentrations between 0.0064nM to 100nM.

### Example 3-4. Quantitative analysis of c-Met mRNA expression in lung cancer cell

The expression rate of c-Met mRNA, whose expression was inhibited by the siRNA liposome complex, was measured by bDNA analysis using Quantigene 2.0 system (Panomics, Inc.).

After treating the human lung cancer cell line with the 10nM siRNA liposome complex for 24 hours, mRNA was quantified. According to manufacturer's protocol, 100µl of a lysis mixture (Panomics, Quantigene 2.0 bDNA kit) was treated per well of 96-well plate to lyze the cells at 50□ for 1 hour. Probe specifically binding to c-Met mRNA (Panomics, Cat.# SA-10157) was purchased from Panomics, Inc., and mixed together with 80µl of the obtained cell sample in a 96 well plate. Reaction was performed at 55□ for 16 to 20 hours so that mRNA could be immobilized in the well and bind to the probe. Subsequently, 100µl of the amplification reagent of the kit was introduced in each well, reacted at 50 and washed, which process was performed in two stages. 100µl of the third amplification reagent was introduced and reacted at 50, and then, 100µl of a luminescence inducing reagent was introduced, and after 5 minutes, luminescence was measured by a microplate reader (Bio-Tek, Synergy-HT) and expressed as percentage of that (100%) of the control which was treated with lipofectamine only. The percentage indicates c-Met mRNA expression rate of each siRNA-treated test group relative to that of the control.

As shown in Table 7, it was confirmed that among 20 kinds of siRNA, 16 kinds of siRNAs inhibit c-Met expression by 40% or less, 1 kind of siRNA inhibits c-Met expression by 40% to 70%, and 3 kinds of siRNAs inhibit c-Met expression by 70% or more.

**[Table 7] Relative expression rate of c-Met mRNA in human lung cancer cell line (A549) treated with 10nM siRNA**

| SEQ ID NO | Sequence (5' -> 3') | siRNA No. | c-Met mRNA expression rate (%) |
|---|---|---|---|
| 2 | GAAAGAGGCACTAGCAAA | 1 | 66.7 |
| 3 | GCACTAGCAAAGTCCGAGA | 2 | 22.7 |
| 4 | CAGCAAAGCCAATTTATCA | 3 | 69.2 |
| 5 | CTATGATGATCAACTCATT | 4 | 81.4 |
| 6 | CAATCATACTGCTGACATA | 5 | 68.5 |
| 7 | CTCTAGATGCTCAGACTTT | 6 | 71.5 |
| 8 | TCTGGATTGCATTCCTACA | 7 | 81.8 |
| 9 | CTGGATTGCATTCCTACAT | 8 | 99.8 |
| 10 | GCACAAAGCAAGCCAGATT | 9 | 84.6 |
| 11 | CTGCTTTAATAGGACACTT | 10 | 68.0 |
| 12 | CAGGTTGTGGTTTCTCGAT | 11 | 68.8 |
| 13 | CTGGTTATCACTGGGAAGA | 12 | 67.1 |
| 14 | TTGGTCCTGCCATGAATAA | 13 | 71.7 |
| 15 | AGACAAGCATCTTCAGTTA | 14 | 55.5 |
| 16 | TCGCTCTAATTCAGAGATA | 15 | 68.1 |
| 17 | TCAGAGATAATCTGTTGTA | 16 | 99.9 |
| 18 | GTGAGAATATACACTTACA | 17 | 12.8 |
| 19 | GGTGTTGTCTCAATATCAA | 18 | 60.5 |
| 20 | CATTTGGATAGGCTTGTAA | 19 | 115.4 |
| 21 | CCAAAGGCATGAAATATCT | 20 | 26.6 |

For the 3 kinds of siRNAs 2, 17 and 20 having excellent gene expression inhibition effect in Table 7, the extent of decreasing c-Met mRNA expression was examined in the range of 100nM to 0.0064nM of siRNAs using A549 cell line to calculate IC₅₀, and the results are described in the following Table 8. The IC₅₀ value was calculated using SofrMax pro software supported by Spectra Max 190 (ELISA equipment) model. Comparing the IC₅₀ values of siRNAs 2, 17 and 20 with those of siRNAs 14 and 15, it can be seen that the siRNAs 2, 17 and 20 show about 5 to 100 time higher inhibition than siRNAs 14 and 15..

**[Table 8] IC₅₀(nM) in A549 cell line**

| siRNA SEQ ID NO | siRNA No. | corresponding mRNA SEQ ID NO. | A549 (IC50 : nM) |
|---|---|---|---|
| 24,25 | 2 | 3 | 0.29 |
| 54,55 | 17 | 18 | 0.87 |
| 60,61 | 20 | 21 | 0.75 |
| 48,49 | 14 | 15 | 4.35 |
| 50,51 | 15 | 16 | 29 |

### Example 3-5. Quantitative analysis of c-Met mRNA expression in liver cancer cell

Liver cancer cell line SK-Hep-1 was respectively treated with each 4nM of siRNAs 2, 17 and 20 of a symmetric structure and siRNAs 21, 22 and 23 of an asymmetric structure with sense strand shorter than antisense strand, which target SEQ ID NO. 3, 18, or 21, and c-Met mRNA inhibition effect was examined, and the results are described in the following Table 9. The experimental method was the same as Examples 3-4.

**[Table 9]**

| siRNA SEQ ID NO | siRNA No. | Structural feature | c-Met expression rate(%) |
|---|---|---|---|
| 24,25 | 2 | Symmetric | 35.7 |
| 62,25 | 21 | Asymmetric | 31.3 |
| 54,55 | 17 | Symmetric | 32.3 |
| 64,55 | 22 | Asymmetric | 43.8 |
| 60,61 | 20 | Symmetric | 40.8 |
| 66, 61 | 23 | Asymmetric | 60.8 |

As shown in the Table 9, if SEQ ID NOs. 3, 18, and 21 are targeted, asymmetric siRNAs could effectively inhibit c-Met expression to a similar degree to symmetric siRNA.

### Example 4. Inhibition test of cell proliferation by siRNA

Cell proliferation inhibition effects by siRNAs 2, 14 and 15 were determined. Human lung cancer cells A549 were seeded in a 96 well plate at the number of 2.5X10³ per well, and after 24 hours, 0.4µl of siRNA liposome complex prepared by the method of Example 3-2 was added to each well as designated concentrations of siRNA according to the method of Example 3-3. 24 hours after the addition, media was replaced with 200µl of fresh cell culture medium, and maintained in a cell incubator under 37°C, 5% CO₂ for 5 days. And then, it was fixed with TCA(Trichloroacetic acid) for 30 minutes and stained with SRB(Sulforhodamine B, Sigma) at room temperature for 30 minutes. The well was washed with 10%(v/v) acetic acid 4~5 times, naturally dried, allowed to stand for one day, and then, 200µl of 10mM unbuffered tris solution (Sigma) was introduced, absorbance was measured at 540nm with a microplate reader (Bio-Tek, Synergy-HT), and expressed as percentage of control (100%) which was treated with Lipofectamine only.

The percentage means cell proliferation rate of the tes group treated with siRNAs 2, 14 or 15 relative to that of the control group. IC₅₀ value was obtained using the percentage value calculated according to the concentration of siRNA treated, and the results are described in the following Table 10. As shown in the Table 10, siRNA 2 exhibits 20~50 time lower IC₅₀ value than siRNAs 14 and 15, thus indicating that cell proliferation inhibition effect of siRNA 2 on cell proliferation is 20~50 time higher than that of siRNAs 14 and 15. Therefore, the siRNA 2 of the present invention may decrease c-Met mRNA expression, and directly induce inhibition of cancer cell proliferation due to the decrease in c-Met expression thus exhibiting extraordinarily excellent anticancer effect.

**[Table 10] Cell division inhibition effect of c-Met-targeting siRNA (A549: IC₅₀(nM))**

| SEQ ID NO | siRNA No. | IC50 (nM) |
|---|---|---|
| 48,49 | 14 | 116 |
| 50,51 | 15 | 50.8 |
| 24,25 | 2 | 2.44 |

### Example 5. Effect of siRNA on immunoactive cytokine release

To evaluate whether or not the siRNA of the present invention has immune toxicity, experiment was conducted according to the following procedures.

### Example 5-1. Preparation of peripheral blood mononuclear cells

Human peripheral blood mononuclear cells (PBMCs) were separated from blood supplied by healthy volunteer at the experiment day using Histopaque 1077 reagent (Sigma, St Louis, MO, USA) by density gradient centrifugation (Boyum A. Separation of leukocytes from blood and bone marrow. Scand J Clin Lab Invest 21 (Suppl97):77, 1968). The blood was carefully introduced on the Histopaque 1077 reagent transferred in a 15ml tube at 1:1 ratio (by weight) so as not to be mixed with each other. After centrifugation at room temperature, 400 x g, for 30 minutes, only the PBMC containing layer was separated with a sterilized pipette. Into the tube containing the separated PBMCs, 10ml of phosphate buffered saline (PBS) was introduced, and then, the mixture was centrifuged at 250 x g for 10 minutes, and PBMCs were additionally washed twice with 5ml of PBS. The separated PBMCs were suspended with serum-free x-vivo 15 medium (Lonza, Walkersville, MD, USA) to a concentration of 4 x 10⁶ cells/ml, and seeded in the volume of 100µl per well in a 96-well plate.

### Example 5-2. Formulation of siRNA- DOTAP complex

A complex of siRNA-DOTAP for transfecting PBMCs prepared in Example 5-1 was prepared as follows. 5ul of a DOTAP transfection reagent (ROCHE, Germany) and 45ul of x-vivo 15 medium, and 1 ul (50 uM) of the siRNA and 49ul of x-vivo 15 medium were respectively mixed, and then, reacted at room temperature for 10 minutes. After 10 minutes, the DOTAP containing solution and the siRNA containing solution were mixed and reacted at a temperature of 20 to 25 for 20 minutes to prepare a siRNA-DOTAP complex.

### Example 5-3. Cell culture

To 100µl of the seeded PBMC culture media, the siRNA-DOTAP complexes of the siRNAs 2, 14 and 15 prepared according to Example 5-2 were respectively added in the volume of 100ul per well (the final concentration of siRNA was 250nM), and then, cultured in a CO₂ incubator of 37 for 18 hours. As control, cell culture groups not treated with the siRNA-DOTAP complex and cell culture groups treated with DOTAP only without siRNA were used. And, Poly I:C (Polyinosinic-polycytidylic acid postassium salt, Sigma, USA) and APOB-1 siRNA (sense GUC AUC ACA CUG AAU ACC AAU (SEQ ID NO 99), antisense : *AUU GGU AUU CAG UGU GAU GAC AC (SEQ ID NO 100), *: 5' phosphates, provided by ST Pharm Co. Ltd.), known to induce an immune response, instead of siRNAwere formulated into a complex with DOTAP by the same method as Example 5-2, and cell culture groups were treated therewith and used as positive control. After culture, only cell supernatant was separated.

### Example 5-4. Measurement of immune activity

The amounts of interferon alpha (INF-α) and interferon gamma (INF- γ), tumor necrosis factor (TNF-α), and interleukin-12 (IL-12) released in the supernatant were measured using Procarta Cytokine assay kit (Affymetrix, USA). Specifically, 50ul of bead to which antibody to cytokine was attached (antibody bead) was transferred to a filter plate and washed with wash buffer once, and then, 50ul of supernatant of the PMBC culture fluid and a cytokine standard solution were added and incubated at room temperature for 60 minutes while shaking at 500rpm.

Then, the solution was washed with washing buffer once, 25ul of detection antibody included in the kit was added, and reacted at room temperature for 30 minutes while shaking at 500rpm. Again, the reaction solution was removed under reduced pressure and washed, and then, 50ul of streptavidin-PE (streptavidin phycoerythrin) included in the kit was added, and reacted at room temperature for 30 minutes while shaking at 500rpm, and then, the reaction solution was removed and washed three times. 120ul of reading buffer was added and the reaction solution was shaken at 500rpm for 5 minutes, and then, PE fluorescence per cytokine bead was measured using Luminex equipment ((Bioplex luminex system, Biorad, USA), and the results are shown in Figs. 1a-1d. The cytokine concentration in the sample was calculated from a standard calibration curve of 1.22~20,000 pg/ml range.

In Figs. 1a-1d, 'Medium' denotes non-treated control, 'DOTAP' denotes only DOTAP-treated group, 'POLY I:C' or 'APOB-1' denotes positive control group, 'siRNA 2' denotes a test group treated with the siRNAs of SEQ ID NOs. 24 and 25, 'siRNA 14' denotes a test group treated with the siRNAs of SEQ ID NOs. 48 and 49, and 'siRNA 15' denotes a test group treated with the siRNA of SEQ ID NOs. 50 and 51. The Figs. 1a-1d shows cytokine level released in the PBMC, wherein 1a denotes interferon alpha, 1b denotes interferon gamma, 1 c denotes interleukin-12, and 1d denotes tumor necrosis factor.

The siRNA 2 exhibited very slight increase in all cytokines compared to control and only DOTAP-only-treated group, and the increase is almost insignificant compared to the increase of cytokine induced by POLY I:C and APOB-1 used as positive control. And, comparing with siRNA 14 and siRNA 15, it can be seen that increase in interferon alpha and interferon gamma, particularly in interferon alpha, is remarkably low. Thus, it was confirmed that the siRNA 2 scarcely induces immune activity in human PBMC.

### Example 6. Preparation of chemically modified siRNA for inhibition of c-Met expression

The siRNAs 2, 17 and 20 prepared in Example 2 were designed so that the chemical structures may be modified in 6 forms (mod1~6) as shown in the above Table 4. The chemically modified siRNA was synthesized by ST Pharm Co. Ltd (Korea). The 17 kinds of siRNAs chemically modified are shown in the following Table 11, wherein the notation of the chemical modification is as explained in the above Table 3.

**[Table 11]**

| SEQ ID NO | Sequence (5'-> 3') | siRNA designation | |
|---|---|---|---|
| 65 | GCACUAGCAAAGUCCGAGAdT*dT | siRNA24 | siRNA 2 -mod1 |
| 66 | UCUCGGACUUUGCUAGUGCdT*dT | | |
| 67 | GCACUAGCAAAGUCCGAGAdT*dT | siRNA25 | siRNA 2 -mod2 |
| 68 | UCUCGGACUUUGCUAGUGCdT*dT | | |
| 69 | GCACUAGCAAAGUCCGAGAdT*dT | siRNA26 | siRNA 2 -mod3 |
| 70 | UCUCGGACUUUGCUAGUGCdT*dT | | |
| 71 | GCACuAGCAAAGuCCGAGAdT*dT | siRNA27 | siRNA 2 -mod4 |
| 72 | UCuCGGACuUUGCuAGuGCdT*dT | | |
| 73 | GCACUAGCAAAGUCCGAGAdT*dT | siRNA28 | siRNA 2 -mod5 |
| 74 | UCUCGGACUUUGCUAGUGCdT*dT | | |
| 75 | GUGAGAAUAUACACUUACAdT*dT | siRNA29 | siRNA 17 -mod1 |
| 76 | UGUAAGUGUAUAUUCUCACdT*dT | | |
| 77 | GUGAGAAUAUACACUUACAdT*dT | siRNA30 | siRNA 17 -mod2 |
| 78 | UGUAAGUGUAUAUUCUCACdT*dT | | |
| 79 | GUGAGAAUAUACACUUACAdT*dT | siRNA31 | siRNA 17 -mod3 |
| 80 | UGUAAGUGUAUAUUCUCACdT*dT | | |
| 81 | GuGAGAAuAuACACuuACAdT*dT | siRNA32 | siRNA 17 -mod4 |
| 82 | UGuAAGuGuAUAuuCuCACdT*dT | | |
| 83 | GUGAGAAUAUACACUUACAdT*dT | siRNA33 | siRNA 17 -mod5 |
| 84 | UGUAAGUGUAUAUUCUCACdT*dT | | |
| 85 | GUGAGAAUAUACACUUACAdT*dT | siRNA34 | siRNA 17 -mod6 |
| 86 | UGUAAGUGUAUAUUCUCACdT*dT | | |
| 87 | CCAAAGGCAUGAAAUAUCUdT*dT | siRNA35 | siRNA 20 -mod1 |
| 88 | AGAUAUUUCAUGCCUUUGGdT*dT | | |
| 89 | CCAAAGGCAUGAAAUAUCUdT*dT | siRNA36 | siRNA 20 -mod2 |
| 90 | AGAUAUUUCAUGCCUUUGGdT*dT | | |
| 91 | CCAAAGGCAUGAAAUAUCUdT*dT | siRNA37 | siRNA 20 -mod3 |
| 92 | AGAUAUUUCAUGCCUUUGGdT*dT | | |
| 93 | CCAAAGGCAuGAAAuAuCudT*dT | siRNA38 | siRNA 20 -mod4 |
| 94 | AGAuAuuuCAUGCCuuuGGdT*dT | | |
| 95 | CCAAAGGCAUGAAAUAUCUdT*dT | siRNA39 | siRNA 20 -mod5 |
| 96 | AGAUAUUUCAUGCCUUUGGdT*dT | | |
| 97 | CCAAAGGCAUGAAAUAUCUdT*dT | siRNA40 | siRNA 20 -mod6 |
| 98 | AGAUAUUUCAUGCCUUUGGdT*dT | | |

### Example 7. Inhibition of c-Met mRNA expression in cancer cell line using chemically modified siRNAs

To confirm whether or not the chemically modified siRNA retains mRNA inhibiting activity in cancer cell line, unmodified siRNA (siRNAs 2, 17 and 20) of Example 2 and 17 siRNAs of siRNAs 24 to 40 chemically modified of Example 6 were respectively formulated into a liposome complex in the same manner as Example 3-2 to transfect human lung cancer cell line (A549, ATCC) (10nM siRNA), the c-Met expression in the transfected cancer cell line was quantitatively analyzed in the same manner as Example 3-4, and the results are described in the following Table 12. In the Table 12, mod0 denotes chemically unmodified siRNA, and ND denotes Not Detected.

**[Table 12] c-Met mRNA relative expression rate (%) in human lung cancer cell line (A549) treated with 10nM of chemically modified siRNA**

| | siRNA 2 | siRNA 17 | siRNA 20 |
|---|---|---|---|
| mod0 | 20.28 | 13.00 | 12.23 |
| mod1 | 18.04 | 38.90 | 44.91 |
| mod2 | 18.74 | 20.70 | 24.61 |
| mod3 | 19.67 | 16.10 | 25.71 |
| mod4 | 34.06 | 22.00 | 60.02 |
| mod5 | 18.76 | 16.60 | 23.06 |
| mod6 | ND | 15.50 | 20.00 |

As shown in the Table 12, even when siRNAs 2, 17 and 20 were chemically modified, the mRNA inhibition effects were retained in cancer cell line. Particularly, mod2, mod3, mod5, and mod6 exhibited effects equivalent to or better than the effect of unmodified siRNA.

### Example 8. inhibition effect of chemically modified siRNA on Immunoactive cytokine release

To investigate the degree of decrease in immune toxicity of siRNA due to chemical modification, siRNAs 2, 17 and 20 were respectively structurally modified to mod1 ― mod6, and then, human peripheral blood mononuclear cells (PBMCs) were treated therewith to quantify released cytokine. The experiment was conducted in the same manner as Example 5, and the concentrations of cytokine (interferon alpha, interferon gamma, interleukin-12, tumor necrosis factor) released from PBMCs in the culture fluid were quantified and shown in the following Table 13. In the Table 13, 'Medium' denotes non-treated control, 'DOTAP' denotes only DOTAP-treated group, 'POLY I:C' or'APOB-1' denotes positive control group, 'siRNA 2' denotes a test group wherein the siRNA 2 is chemically modified with mod0~5, and 'siRNA 20' denotes a test group wherein the siRNA 20 is chemically modified with mod0~6. The mod0 denotes chemically unmodified siRNA, and mod1~6 are as explained in the Table 4.

**[Table 13] Concentration (pg/ml) of cytokine released in cell culture fluid when PBMCs were treated with 250nM of chemically structurally modified siRNA**

| | | INF-alpha | INF-gamma | IL-12p 40 | TNF-alpha |
|---|---|---|---|---|---|
| | MEDIUM | <1.2 | 10.9 | 15 | 32.6 |
| | DOTAP | 9.1 | 18.3 | 43.4 | 131.0 |
| | siApoB-1 | 690.7 | - | - | - |
| | POLY I:C | - | 46.9 | 398.3 | 2691.5 |
| siRNA 2 | mod0 | 6.0 | 6.3 | 45.3 | 96.8 |
| | mod1 | 11.1 | 6.3 | 65.8 | 128.2 |
| | mod2 | 21.4 | 50.5 | 75.2 | 154.1 |
| | mod3 | 8.7 | 7.3 | 67.3 | 124.9 |
| | mod4 | 7.8 | 11.8 | 54.6 | 94.2 |
| | mod5 | 7.8 | 8.3 | 73.6 | 147.4 |
| siRNA 17 | mod0 | 1091.3 | 21.5 | 29.8 | 146.0 |
| | mod1 | 413.2 | 16.3 | 30.0 | 136.9 |
| | mod2 | 23.9 | 11.8 | 88.8 | 181.0 |
| | mod3 | 4.0 | 10.1 | 78.0 | 140.1 |
| | mod4 | 7.0 | 8.3 | 59.1 | 93.9 |
| | mod5 | 60.3 | 10.1 | 59.1 | 147.4 |
| | mod6 | 10.1 | 1.9 | 20.1 | 84.9 |
| siRNA 20 | mod0 | 597.7 | 16.6 | 37.5 | 136.3 |
| | mod1 | 6.0 | 10.1 | 57.4 | 153.6 |
| | mod2 | 6.5 | 14.9 | 61.0 | 108.5 |
| | mod3 | 8.7 | 8.3 | 60.5 | 115.6 |
| | mod4 | 6.5 | 10.1 | 53.8 | 87.0 |
| | mod5 | 23.9 | 13.4 | 73.1 | 161.6 |
| | mod6 | 21.4 | 1.9 | 27.9 | 103.9 |

As shown in the Table 13, the siRNA 2 exhibited no change or very slight increase in all cytokines, compared to the control and only DOTAP-only-treated group.

Meanwhile, the siRNAs 17 and 20 exhibited rapid decrease in interferon alpha due to the chemical modification. For the other cytokines, there is no significant change or very slight increase. Thus, it was confirmed that the chemical modification of the siRNAs 17 and 20 may remarkably decrease immune activity.

### Example 9. Inhibition of off-target effect by sense strand of chemically modified siRNA

The following experiment was conducted to examine whether or not off-target effect by sense strand may be removed through chemical modification of siRNA.

### Example 9-1. Preparation of firefly luciferase vector

A sequence complementary to an antisense strand and a sequence complementary to a sense strand of siRNA were respectively cloned in a pMIR-REPORT(Ambion) vector expressing firefly luciferase to prepare two different plasmids. The complementary sequences were designed and synthesized by Cosmo Genetech such that both ends had Spel and HindIII restriction sites overhang, and then, cloned using Spel and HindIII restriction sites of a pMIR-REPORT vector.

### Example 9-2. Measurement of inhibition of off-target effect through chemical modification of siRNA

Using plasmids comprising respective sequences complementary to each sense strand and antisense strand of siRNA, prepared in Example 9-1, effects of the antisense and sense strands of siRNA were measured. The degree of off-target effect by sense strand can be seen by confirming that if a sense strand binds to RISC and acts on a sequence having a base sequence complementary to the sense strand, the amount of luciferase expressed by firefly Luciferase plasmid having a sequence complementary to the sense strand decreases compared to the cell that is not treated with the siRNA. And, for cells treated with firefly luciferase plasmid having a sequence complementary to antisense, the degree of retention of siRNA activity by antisense after chemical modification may be confirmed by degree of reduction in luciferase exhibited by the siRNA.

Specifically, the firefly luciferase vector prepared in Example 9-1 was transfected in HeLa and A549 cells (ATCC) together with the siRNA, and then, the amount of expressed firefly luciferase was measured by luciferase assay. One day before transfection, the HeLa and A549 cell lines were prepared in a 24 well plate at 6*10⁴ cells/well. The luciferase vector (100ng) in which complementary base sequences were cloned were transfected in Opti-MEM medium (Gibco) using lipofectamine 2000 (Invitrogen) together with a vector for normalization, pRL-SV40 vector (2ng, Promega) expressing renilla luciferase. After 24 hours, the cells were lyzed using passive lysis buffer (Promega), and then, luciferase activity was measured by dual luciferase assay kit (Promega).

The measured firefly luciferase value was normalized for transfection efficiency with the measured renilla luciferase value, and then, percentage value to the normalized luciferase value (100%) of control, which was transfected with renilla luciferase vector and firefly luciferase vector in which sequences complementary to each strand were cloned without siRNA, was calculated and described in the following Table 14. In the Table 14, mod0 denotes chemically unmodified siRNA, and model~6 are as explained in the Table 4.

**[Table14] Off-target effect decrease through chemical modification of siRNA**

| siRNA No. | Name of chemically modified structure | Luciferase activity(%) | | | |
|---|---|---|---|---|---|
| | | HeLa | | A549 | |
| | | Plasmid comprising sequence complementary to sense strand | Plasmid comprising sequence complementar y to antisense strand | Plasmid comprising sequence complementary to sense strand | Plasmid comprising sequence complementar y to antisense strand |
| 2 | mod0 | 118.4 | 9.1 | 139.3 | 5.9 |
| 20 | mod0 | 21.08 | 7.68 | 17.56 | 7.08 |
| | mod1 | 8.19 | 30.29 | 9.6 | 65.01 |
| | mod2 | 48.38 | 12.45 | 80.91 | 26.14 |
| | mod3 | 31.34 | 19.03 | 38.23 | 15.81 |
| | mod4 | 12.23 | 47.58 | 16.27 | 56.91 |
| | mod5 | 56.73 | 8.14 | 63.49 | 17.64 |

As shown in the Table 14, in human lung cancer cell line A549 and uterine cervical cancer cell line HeLa, unmodified siRNA (mod0) per se had no off-target effect by sense strand in case of siRNA 2. However, in the case of siRNA 20, slight off-target effect by sense strand was seen through decrease in the activity of firefly luciferase having sequence complementary to the sense strand, but if chemically modified, sense strand effect was decreased and antisense effect was maintained, particularly in mod2 and 5.

## Claims

1. A double stranded siRNA (small interfering RNA) of 15 to 30 bp, which targets an mRNA region corresponding to at least one selected from c-Met cDNA regions described in the following Table 1-1:
**[Table 1-1]**
| Sequence No. | Sequence (5' -> 3') |
|---|---|
| 2 | GAAAGAGGCACTAGCAAA |
| 3 | GCACTAGCAAAGTCCGAGA |
| 4 | CAGCAAAGCCAATTTATCA |
| 5 | CTATGATGATCAACTCATT |
| 6 | CAATCATACTGCTGACATA |
| 7 | CTCTAGATGCTCAGACTTT |
| 8 | TCTGGATTGCATTCCTACA |
| 9 | CTGGATTGCATTCCTACAT |
| 10 | GCACAAAGCAAGCCAGATT |
| 11 | CTGCTTTAATAGGACACTT |
| 12 | CAGGTTGTGGTTTCTCGAT |
| 13 | CTGGTTATCACTGGGAAGA |
| 14 | TTGGTCCTGCCATGAATAA |
| 15 | AGACAAGCATCTTCAGTTA |
| 16 | TCGCTCTAATTCAGAGATA |
| 17 | TCAGAGATAATCTGTTGTA |
| 18 | GTGAGAATATACACTTACA |
| 19 | GGTGTTGTCTCAATATCAA |
| 20 | CATTTGGATAGGCTTGTAA |
| 21 | CCAAAGGCATGAAATATCT |

2. The siRNA according to claim 1, wherein the siRNA targets an mRNA region corresponding to at least one base sequence selected from the group consisting of SEQ ID NOs 3, 18 and 21.

3. The siRNA according to claim 1, wherein the siRNA comprises an overhang consisting of 1 to 5 nucleotides at 3' end, 5' end, or both ends.

4. The siRNA according to claim 2, wherein the siRNA comprises nucleotide sequence selected from the group consisting of siRNAs 1 to 23 described in the following Table 6:
**[Table 6]**
| Sequence No. | Sequence (5' -> 3') | Strand | siRNA designation |
|---|---|---|---|
| 22 | GUAAAGAGGCACUAGCAAAdTdT | Sense | siRNA 1 |
| 23 | UUUGCUAGUGCCUCUUUACdTdT | Antisense | |
| 24 | GCACUAGCAAAGUCCGAGAdTdT | Sense | siRNA 2 |
| 25 | UCUCGGACUUUGCUAGUGCdTdT | Antisense | |
| 26 | CAGCAAAGCCAAUUUAUCAdTdT | Sense | siRNA 3 |
| 27 | UGAUAAAUUGGCUUUGCUGdTdT | Antisense | |
| 28 | CUAUGAUGAUCAACUCAUUdTdT | Sense | siRNA 4 |
| 29 | AAUGAGUUGAUCAUCAUAGdTdT | Antisense | |
| 30 | CAAUCAUACUGCUGACAUAdTdT | Sense | siRNA 5 |
| 31 | UAUGUCAGCAGUAUGAUUGdTdT | Antisense | |
| 32 | CUCUAGAUGCUCAGACUUUdTdT | Sense | siRNA 6 |
| 33 | AAAGUCUGAGCAUCUAGAGdTdT | Antisense | |
| 34 | UCUGGAUUGCAUUCCUACAdTdT | Sense | siRNA 7 |
| 35 | UGUAGGAAUGCAAUCCAGAdTdT | Antisense | |
| 36 | CUGGAUUGCAUUCCUACAUdTdT | Sense | siRNA 8 |
| 37 | AUGUAGGAAUGCAAUCCAGdTdT | Antisense | |
| 38 | GCACAAAGCAAGCCAGAUUdTdT | Sense | siRNA 9 |
| 39 | AAUCUGGCUUGCUUUGUGCdTdT | Antisense | |
| 40 | CUGCUUUAAUAGGACACUUdTdT | Sense | siRNA 10 |
| 41 | AAGUGUCCUAUUAAAGCAGdTdT | Antisense | |
| 42 | CAGGUUGUGGUUUCUCGAUdTdT | Sense | siRNA 11 |
| 43 | AUCGAGAAACCACAACCUGdTdT | Antisense | |
| 44 | CUGGUUAUCACUGGGAAGAdTdT | Sense | siRNA 12 |
| 45 | UCUUCCCAGUGAUAACCAGdTdT | Antisense | |
| 46 | UUGGUCCUGCCAUGAAUAAdTdT | Sense | siRNA 13 |
| 47 | UUAUUCAUGGCAGGACCAAdTdT | Antisense | |
| 48 | AGACAAGCAUCUUCAGUUAdTdT | Sense | siRNA 14 |
| 49 | UAACUGAAGAUGCUUGUCUdTdT | Antisense | |
| 50 | UCGCUCUAAUUCAGAGAUAdTdT | Sense | siRNA 15 |
| 51 | UAUCUCUGAAUUAGAGCGAdTdT | Antisense | |
| 52 | UCAGAGAUAAUCUGUUGUAdTdT | Sense | siRNA 16 |
| 53 | UACAACAGAUUAUCUCUGAdTdT | Antisense | |
| 54 | GUGAGAAUAUACACUUACAdTdT | Sense | siRNA 17 |
| 55 | UGUAAGUGUAUAUUCUCACdTdT | Antisense | |
| 56 | GGUGUUGUCUCAAUAUCAAdTdT | Sense | siRNA 18 |
| 57 | UUGAUAUUGAGACAACACCdTdT | Antisense | |
| 58 | CAUUUGGAUAGGCUUGUAAdTdT | Sense | siRNA 19 |
| 59 | UUACAAGCCUAUCCAAAUGdTdT | Antisense | |
| 60 | CCAAAGGCAUGAAAUAUCUdTdT | Sense | siRNA 20 |
| 61 | AGAUAUUUCAUGCCUUUGGdTdT | Antisense | |
| 62 | CUAGCAAAGUCCGAGA | Sense | siRNA 21 |
| 25 | UCUCGGACUUUGCUAGUGCdTdT | Antisense | |
| 63 | AGAAUAUACACUUACA | Sense | siRNA 22 |
| 55 | UGUAAGUGUAUAUUCUCACdTdT | Antisense | |
| 64 | GAUUGCAUUCCUACAU | Sense | siRNA 23 |
| 61 | AGAUAUUUCAUGCCUUUGGdTdT | Antisense | |

5. The siRNA according to claim 4, wherein the siRNA is selected from the group consisting of
siRNA 2 comprising a sense sequence of SEQ ID NO 24 and an antisense sequence of SEQ ID NO 25;
siRNA 17 comprising a sense sequence of SEQ ID NO 54 and an antisense sequence of SEQ ID NO 55;
siRNA 20 comprising a sense sequence of SEQ ID NO 60 and an antisense sequence of SEQ ID NO 61;
siRNA 21 comprising a sense sequence of SEQ ID NO 62 and an antisense sequence of SEQ ID NO 25;
siRNA 22 comprising a sense sequence of SEQ ID NO 63 and an antisense sequence of SEQ ID NO 55; and
siRNA 23 comprising a sense sequence of SEQ ID NO 64 and an antisense sequence of SEQ ID NO 61.

6. The siRNA according to claim 1, wherein the sugar or base structure of at least one ribonucleotide, or a linkage between the ribonucleotides is chemically modified.

7. The siRNA according to claim 6, wherein the chemical modification is modification of a phosphodiester linkage at 3' end, 5' end or both ends with a boranophosphate or a phosphorothioate linkage.

8. The siRNA according to claim 6, wherein the chemical modification is introduction of ENA(Ethylene bridge nucleic acid) at 3' end, 5' end, or both ends.

9. The siRNA according to claim 6, wherein the chemical modification is substitution of 2'―OH (hydroxyl group) of the ribose ring with at least one selected from the group consisting of -NH₂(amino group), -C-allyl group, -F(fluoro group), and -O-Me(methyl group).

10. The siRNA according to claim 6, wherein the chemically modified siRNA comprises nucleotide sequence selected from the group consisting of siRNA 24 to 40 described in the following Table 11-1.
**[Table 11-1]**
| Sequence No. | Sequence (5'-> 3') | siRNA designation |
|---|---|---|
| 65 | GCACUAGCAAAGUCCGAGAdT*dT | siRNA24 |
| 66 | UCUCGGACUUUGCUAGUGCdT*dT | |
| 67 | GCACUAGCAAAGUCCGAGAdT*dT | siRNA25 |
| 68 | UCUCGGACUUUGCUAGUGCdT*dT | |
| 69 | GCACUAGCAAAGUCCGAGAdT*dT | siRNA26 |
| 70 | UCUCGGACUUUGCUAGUGCdT*dT | |
| 71 | GCACuAGCAAAGuCCGAGAdT*dT | siRNA27 |
| 72 | UCuCGGACuUUGCuAGuGCdT*dT | |
| 73 | GCACUAGCAAAGUCCGAGAdT*dT | siRNA28 |
| 74 | UCUCGGACUUUGCUAGUGCdT*dT | |
| 75 | GUGAGAAUAUACACUUACAdT*dT | siRNA29 |
| 76 | UGUAAGUGUAUAUUCUCACdT*dT | |
| 77 | GUGAGAAUAUACACUUACAdT*dT | siRNA30 |
| 78 | UGUAAGUGUAUAUUCUCACdT*dT | |
| 79 | GUGAGAAUAUACACUUACAdT*dT | siRNA31 |
| 80 | UGUAAGUGUAUAUUCUCACdT*dT | |
| 81 | GuGAGAAuAuACACuuACAdT*dT | siRNA32 |
| 82 | UGuAAGuGuAUAuuCuCACdT*dT | |
| 83 | GUGAGAAUAUACACUUACAdT*dT | siRNA33 |
| 84 | UGUAAGUGUAUAUUCUCACdT*dT | |
| 85 | GUGAGAAUAUACACUUACAdT*dT | siRNA34 |
| 86 | UGUAAGUGUAUAUUCUCACdT*dT | |
| 87 | CCAAAGGCAUGAAAUAUCUdT*dT | siRNA35 |
| 88 | AGAUAUUUCAUGCCUUUGGdT*dT | |
| 89 | CCAAAGGCAUGAAAUAUCUdT*dT | siRNA36 |
| 90 | AGAUAUUUCAUGCCUUUGGdT*dT | |
| 91 | CCAAAGGCAUGAAAUAUCUdT*dT | siRNA37 |
| 92 | AGAUAUUUCAUGCCUUUGGdT*dT | |
| 93 | CCAAAGGCAuGAAAuAuCudT*dT | siRNA38 |
| 94 | AGAuAuuuCAUGCCuuuGGdT*dT | |
| 95 | CCAAAGGCAUGAAAUAUCUdT*dT | siRNA39 |
| 96 | AGAUAUUUCAUGCCUUUGGdT*dT | |
| 97 | CCAAAGGCAUGAAAUAUCUdT*dT | siRNA40 |
| 98 | AGAUAUUUCAUGCCUUUGGdT*dT | |
In the above Table 11-1, notation of chemical modification is as described in the following Table 3:
**[Table 3]**
| notation | Introduced chemical modification |
|---|---|
| * | Substitution of a phosphodiester linkage with a phosphorothioate linkage |
| underline | Substitution of 2'-OH of the ribose ring with 2'-O-Me |
| Lower case letter | Substitution of 2'-OH of the ribose ring with 2'-F |
| Bold letter | Introduction of ENA(ethylene bridge nucleic acid) |

11. An expression vector comprising the siRNA according to any one of claims 1 to 10.

12. The expression vector according to claim 11, wherein the expression vector is selected from the group consisting of a plasmid, an adeno-associated virus vector, a retrovirus vector, a vaccinia virus vector, and an oncolytic adenovirus vector.

13. An anticancer composition containing the siRNA according to any one of claims 1 to10 as an active ingredient.

14. The anticancer composition according to claim 13, comprising the siRNA in the form of a complex with a nucleic acid delivery system.

15. The anticancer composition according to claim 14, wherein the nucleic acid delivery system is selected from the group consisting of a viral vector, a non-viral vector, liposome, cationic polymer, micelle, emulsion, and solid lipid nanoparticles.

16. The anticancer composition according to claim 13, further comprising anticancer chemotherapeutics, or
siRNA for inhibiting the expression of one selected from the group consisting of growth factor, growth factor receptor, downstream signal transduction protein, viral oncogene, and anticancer agent resistant gene.

17. A method for inhibiting synthesis and/or expression of c-Met, comprising
preparing the siRNA according to any one of claim 1 to claim 10; and contacting the siRNA with c-Met-expressing cells.

18. A method for inhibiting growth of cancer cells, comprising
preparing the siRNA according to any one of claim 1 to claim 10; and contacting the siRNA with c-Met-expressing cancer cells.

19. A method for preventing and/or treating cancer, comprising
preparing the siRNA according to any one of claim 1 to claim 10; and administering the siRNA to a patient in a therapeutically effective amount.


<110>    SAMYANG CORPORATION

<120>    siRNA for inhibition against c-Met expression and anticancer
         composition containing the same

<130>    OPP-2010-3919-KR

<150>    KR10-2009-0135665
<151>    2009-12-31

<160>    100

<170>    KopatentIn 1.71

<210>    1
<211>    6641
<212>    DNA
<213>    Artificial Sequence

<220>
<223>    NM_000245 Homo sapiens met proto-oncogene (hepatocyte growth
         factor receptor)(MET), transcript variant 2, mRNA

<400>    1
gccctcgccg cccgcggcgc cccgagcgct ttgtgagcag atgcggagcc gagtggaggg      60

cgcgagccag atgcggggcg acagctgact tgctgagagg aggcggggag gcgcggagcg     120

cgcgtgtggt ccttgcgccg ctgacttctc cactggttcc tgggcaccga aagataaacc     180

tctcataatg aaggcccccg ctgtgcttgc acctggcatc ctcgtgctcc tgtttacctt     240

ggtgcagagg agcaatgggg agtgtaaaga ggcactagca aagtccgaga tgaatgtgaa     300

tatgaagtat cagcttccca acttcaccgc ggaaacaccc atccagaatg tcattctaca     360

tgagcatcac attttccttg gtgccactaa ctacatttat gttttaaatg aggaagacct     420

tcagaaggtt gctgagtaca agactgggcc tgtgctggaa cacccagatt gtttcccatg     480

tcaggactgc agcagcaaag ccaatttatc aggaggtgtt tggaaagata acatcaacat     540

ggctctagtt gtcgacacct actatgatga tcaactcatt agctgtggca gcgtcaacag     600

agggacctgc cagcgacatg tctttcccca caatcatact gctgacatac agtcggaggt     660

tcactgcata ttctccccac agatagaaga gcccagccag tgtcctgact gtgtggtgag     720

cgccctggga gccaaagtcc tttcatctgt aaaggaccgg ttcatcaact tctttgtagg     780

caataccata aattcttctt atttcccaga tcatccattg cattcgatat cagtgagaag 840

gctaaaggaa acgaaagatg gttttatgtt tttgacggac cagtcctaca ttgatgtttt 900

acctgagttc agagattctt accccattaa gtatgtccat gcctttgaaa gcaacaattt 960

tatttacttc ttgacggtcc aaagggaaac tctagatgct cagacttttc acacaagaat 1020

aatcaggttc tgttccataa actctggatt gcattcctac atggaaatgc ctctggagtg 1080

tattctcaca gaaaagagaa aaaagagatc cacaaagaag gaagtgttta atatacttca 1140

ggctgcgtat gtcagcaagc ctggggccca gcttgctaga caaataggag ccagcctgaa 1200

tgatgacatt cttttcgggg tgttcgcaca aagcaagcca gattctgccg aaccaatgga 1260

tcgatctgcc atgtgtgcat tccctatcaa atatgtcaac gacttcttca acaagatcgt 1320

caacaaaaac aatgtgagat gtctccagca tttttacgga cccaatcatg agcactgctt 1380

taataggaca cttctgagaa attcatcagg ctgtgaagcg cgccgtgatg aatatcgaac 1440

agagtttacc acagctttgc agcgcgttga cttattcatg ggtcaattca gcgaagtcct 1500

cttaacatct atatccacct tcattaaagg agacctcacc atagctaatc ttgggacatc 1560

agagggtcgc ttcatgcagg ttgtggtttc tcgatcagga ccatcaaccc ctcatgtgaa 1620

ttttctcctg gactcccatc cagtgtctcc agaagtgatt gtggagcata cattaaacca 1680

aaatggctac acactggtta tcactggaaa gaagatcacg aagatcccat tgaatggctt 1740

gggctgcaga catttccagt cctgcagtca atgcctctct gccccaccct ttgttcagtg 1800

tggctggtgc cacgacaaat gtgtgcgatc ggaggaatgc ctgagcggga catggactca 1860

acagatctgt ctgcctgcaa tctacaaggt tttcccaaat agtgcacccc ttgaaggagg 1920

gacaaggctg accatatgtg gctgggactt tggatttcgg aggaataata aatttgattt 1980

aaagaaaact agagttctcc ttggaaatga gagctgcacc ttgactttaa gtgagagcac 2040

gatgaataca ttgaaatgca cagttggtcc tgccatgaat aagcatttca atatgtccat 2100

aattatttca aatggccacg ggacaacaca atacagtaca ttctcctatg tggatcctgt 2160

aataacaagt atttcgccga aatacggtcc tatggctggt ggcactttac ttactttaac 2220

tggaaattac ctaaacagtg ggaattctag acacatttca attggtggaa aaacatgtac 2280

tttaaaaagt gtgtcaaaca gtattcttga atgttatacc ccagcccaaa ccatttcaac 2340

tgagtttgct gttaaattga aaattgactt agccaaccga gagacaagca tcttcagtta 2400

ccgtgaagat cccattgtct atgaaattca tccaaccaaa tcttttatta gtggtgggag 2460

cacaataaca ggtgttggga aaaacctgaa ttcagttagt gtcccgagaa tggtcataaa 2520

tgtgcatgaa gcaggaagga actttacagt ggcatgtcaa catcgctcta attcagagat 2580

aatctgttgt accactcctt ccctgcaaca gctgaatctg caactccccc tgaaaaccaa 2640

agcctttttc atgttagatg ggatcctttc caaatacttt gatctcattt atgtacataa 2700

tcctgtgttt aagccttttg aaaagccagt gatgatctca atgggcaatg aaaatgtact 2760

ggaaattaag ggaaatgata ttgaccctga agcagttaaa ggtgaagtgt taaaagttgg 2820

aaataagagc tgtgagaata tacacttaca ttctgaagcc gttttatgca cggtccccaa 2880

tgacctgctg aaattgaaca gcgagctaaa tatagagtgg aagcaagcaa tttcttcaac 2940

cgtccttgga aaagtaatag ttcaaccaga tcagaatttc acaggattga ttgctggtgt 3000

tgtctcaata tcaacagcac tgttattact acttgggttt ttcctgtggc tgaaaaagag 3060

aaagcaaatt aaagatctgg gcagtgaatt agttcgctac gatgcaagag tacacactcc 3120

tcatttggat aggcttgtaa gtgcccgaag tgtaagccca actacagaaa tggtttcaaa 3180

tgaatctgta gactaccgag ctacttttcc agaagatcag tttcctaatt catctcagaa 3240

cggttcatgc cgacaagtgc agtatcctct gacagacatg tcccccatcc taactagtgg 3300

ggactctgat atatccagtc cattactgca aaatactgtc cacattgacc tcagtgctct 3360

aaatccagag ctggtccagg cagtgcagca tgtagtgatt gggcccagta gcctgattgt 3420

gcatttcaat gaagtcatag gaagagggca ttttggttgt gtatatcatg ggactttgtt 3480

ggacaatgat ggcaagaaaa ttcactgtgc tgtgaaatcc ttgaacagaa tcactgacat 3540

aggagaagtt tcccaatttc tgaccgaggg aatcatcatg aaagatttta gtcatcccaa 3600

tgtcctctcg ctcctgggaa tctgcctgcg aagtgaaggg tctccgctgg tggtcctacc 3660

atacatgaaa catggagatc ttcgaaattt cattcgaaat gagactcata atccaactgt 3720

aaaagatctt attggctttg gtcttcaagt agccaaaggc atgaaatatc ttgcaagcaa 3780

aaagtttgtc cacagagact tggctgcaag aaactgtatg ctggatgaaa aattcacagt 3840

caaggttgct gattttggtc ttgccagaga catgtatgat aaagaatact atagtgtaca 3900

caacaaaaca ggtgcaaagc tgccagtgaa gtggatggct ttggaaagtc tgcaaactca 3960

aaagtttacc accaagtcag atgtgtggtc ctttggcgtg ctcctctggg agctgatgac 4020

aagaggagcc ccaccttatc ctgacgtaaa cacctttgat ataactgttt acttgttgca 4080

agggagaaga ctcctacaac ccgaatactg cccagacccc ttatatgaag taatgctaaa 4140

atgctggcac cctaaagccg aaatgcgccc atcctttctt gaactggtgt cccggatatc 4200

agcgatcttc tctactttca ttggggagca ctatgtccat gtgaacgcta cttatgtgaa 4260

cgtaaaatgt gtcgctccgt atccttctct gttgtcatca gaagataacg ctgatgatga 4320

ggtggacaca cgaccagcct ccttctggga gacatcatag tgctagtact atgtcaaagc 4380

aacagtccac actttgtcca atggtttttt cactgcctga cctttaaaag gccatcgata 4440

ttctttgctc ttgccaaaat tgcactatta taggacttgt attgttattt aaattactgg 4500

attctaagga atttcttatc tgacagagca tcagaaccag aggcttggtc ccacaggcca 4560

cggaccaatg gcctgcagcc gtgacaacac tcctgtcata ttggagtcca aaacttgaat 4620

tctgggttga atttttaaaa aatcaggtac cacttgattt catatgggaa attgaagcag 4680

gaaatattga gggcttcttg atcacagaaa actcagaaga gatagtaatg ctcaggacag 4740

gagcggcagc cccagaacag gccactcatt tagaattcta gtgtttcaaa acacttttgt 4800

gtgttgtatg gtcaataaca tttttcatta ctgatggtgt cattcaccca ttaggtaaac 4860

attccctttt aaatgtttgt ttgtttttg agacaggatc tcactctgtt gccagggctg 4920

tagtgcagtg gtgtgatcat agctcactgc aacctccacc tcccaggctc aagcctcccg 4980

aatagctggg actacaggcg cacaccacca tccccggcta atttttgtat tttttgtaga 5040

gacggggttt tgccatgttg ccaaggctgg tttcaaactc ctggactcaa gaaatccacc 5100

cacctcagcc tcccaaagtg ctaggattac aggcatgagc cactgcgccc agcccttata 5160

aatttttgta tagacattcc tttggttgga agaatattta taggcaatac agtcaaagtt 5220

tcaaaatagc atcacacaaa acatgtttat aaatgaacag gatgtaatgt acatagatga 5280

cattaagaaa atttgtatga aataatttag tcatcatgaa atatttagtt gtcatataaa 5340

aacccactgt ttgagaatga tgctactctg atctaatgaa tgtgaacatg tagatgtttt 5400

gtgtgtattt ttttaaatga aaactcaaaa taagacaagt aatttgttga taaatatttt 5460

taaagataac tcagcatgtt tgtaaagcag gatacatttt actaaaaggt tcattggttc 5520

caatcacagc tcataggtag agcaaagaaa gggtggatgg attgaaaaga ttagcctctg 5580

tctcggtggc aggttcccac ctcgcaagca attggaaaca aaacttttgg ggagttttat 5640

tttgcattag ggtgtgtttt atgttaagca aaacatactt tagaaacaaa tgaaaaaggc 5700

aattgaaaat cccagctatt tcacctagat ggaatagcca ccctgagcag aactttgtga 5760

tgcttcattc tgtggaattt tgtgcttgct actgtatagt gcatgtggtg taggttactc 5820

taactggttt tgtcgacgta aacatttaaa gtgttatatt ttttataaaa atgtttattt 5880

ttaatgatat gagaaaaatt ttgttaggcc acaaaaacac tgcactgtga acattttaga 5940

aaaggtatgt cagactggga ttaatgacag catgatttc aatgactgta aattgcgata 6000

aggaaatgta ctgattgcca atacacccca ccctcattac atcatcagga cttgaagcca 6060

agggttaacc cagcaagcta caaagagggt gtgtcacact gaaactcaat agttgagttt 6120

ggctgttgtt gcaggaaaat gattataact aaaagctctc tgatagtgca gagacttacc 6180

agaagacaca aggaattgta ctgaagagct attacaatcc aaatattgcc gtttcataaa 6240

tgtaataagt aatactaatt cacagagtat tgtaaatggt ggatgacaaa agaaaatctg 6300

ctctgtggaa agaaagaact gtctctacca gggtcaagag catgaacgca tcaatagaaa 6360

gaactcgggg aaacatccca tcaacaggac tacacacttg tatatacatt cttgagaaca 6420

ctgcaatgtg aaaatcacgt ttgctattta taaacttgtc cttagattaa tgtgtctgga 6480

cagattgtgg gagtaagtga ttcttctaag aattagatac ttgtcactgc ctatacctgc 6540

agctgaactg aatggtactt cgtatgttaa tagttgttct gataaatcat gcaattaaag 6600

taaagtgatg caacatcttg taaaaaaaaa aaaaaaaaaa a 6641

<210> 2
<211> 19
<212> DNA
<213> Artificial Sequence

<220>
<223> c-Met cDNA sequence started from 77th nucleotide in c-Met gene

<400> 2
gtaaagaggc actagcaaa 19

<210> 3
<211> 19
<212> DNA
<213> Artificial Sequence

<220>
<223> c-Met cDNA sequence started from 86th nucleotide in c-Met gene

<400> 3
gcactagcaa agtccgaga 19

<210> 4
<211> 19
<212> DNA
<213> Artificial Sequence

<220>
<223> c-Met cDNA sequence started from 306th nucleotide in c-Met gene

<400> 4
cagcaaagcc aatttatca 19

<210> 5
<211> 19
<212> DNA
<213> Artificial Sequence

<220>
<223> c-Met cDNA sequence started from 375th nucleotide in c-Met gene

<400> 5
ctatgatgat caactcatt 19

<210> 6
<211> 19
<212> DNA
<213> Artificial Sequence

<220>

<223>    c-Met cDNA sequence started from 444th nucleotide in c-Met gene

<400>    6
caatcatact gctgacata                                                    19

<210>    7
<211>    19
<212>    DNA
<213>    Artificial Sequence

<220>
<223>    c-Met cDNA sequence started from 803rd nucleotide in c-Met gene

<400>    7
ctctagatgc tcagacttt                                                    19

<210>    8
<211>    19
<212>    DNA
<213>    Artificial Sequence

<220>
<223>    c-Met cDNA sequence started from 856th nucleotide in c-Met gene

<400>    8
tctggattgc attcctaca                                                    19

<210>    9
<211>    19
<212>    DNA
<213>    Artificial Sequence

<220>
<223>    c-Met cDNA sequence started from 857th nucleotide in c-Met gene

<400>    9
ctggattgca ttcctacat                                                    19

<210>    10
<211>    19
<212>    DNA
<213>    Artificial Sequence

<220>
<223>    c-Met cDNA sequence started from 1039th nucleotide in c-Met gene

<400>    10
gcacaaagca agccagatt                                                    19

<210>    11
<211>    19
<212>    DNA
<213>    Artificial Sequence

<220>
<223>    c-Met cDNA sequence started from 1188th nucleotide in c-Met gene

<400>    11
ctgctttaat aggacactt                                                    19

<210>    12
<211>    19
<212>    DNA
<213>    Artificial Sequence

<220>
<223>    c-Met cDNA sequence started from 1390th nucleotide in c-Met gene

<400>    12
caggttgtgg tttctcgat                                                    19

<210>    13
<211>    19
<212>    DNA
<213>    Artificial Sequence

<220>
<223>    c-Met cDNA sequence started from 1507th nucleotide in c-Met gene

<400>    13
ctggttatca ctgggaaga                                                    19

<210>    14
<211>    19
<212>    DNA
<213>    Artificial Sequence

<220>
<223>    c-Met cDNA sequence started from 1877th nucleotide in c-Met gene

<400>    14
ttggtcctgc catgaataa                                                    19

<210>    15
<211>    19
<212>    DNA
<213>    Artificial Sequence

<220>
<223>    c-Met cDNA sequence started from 2195th nucleotide in c-Met gene

<400>    15
agacaagcat cttcagtta                                                    19

<210>    16
<211>    19
<212>    DNA
<213>    Artificial Sequence

<220>
<223>    c-Met cDNA sequence started from 2376th nucleotide in c-Met gene

<400>    16
tcgctctaat tcagagata                                                    19

<210>    17
<211>    19
<212>    DNA
<213>    Artificial Sequence

<220>
<223>    c-Met cDNA sequence started from 2386th nucleotide in c-Met gene

<400>    17
tcagagataa tctgttgta                                                    19

<210>    18
<211>    19
<212>    DNA

<213>    Artificial Sequence

<220>
<223>    c-Met cDNA sequence started from 2645th nucleotide in c-Met gene

<400>    18
gtgagaatat acacttaca                                                    19

<210>    19
<211>    19
<212>    DNA
<213>    Artificial Sequence

<220>
<223>    c-Met cDNA sequence started from 2809th nucleotide in c-Met gene

<400>    19
ggtgttgtct caatatcaa                                                    19

<210>    20
<211>    19
<212>    DNA
<213>    Artificial Sequence

<220>
<223>    c-Met cDNA sequence started from 2935th nucleotide in c-Met gene

<400>    20
catttggata ggcttgtaa                                                    19

<210>    21
<211>    19
<212>    DNA
<213>    Artificial Sequence

<220>
<223>    c-Met cDNA sequence started from 3566th nucleotide in c-Met gene

<400>    21
ccaaaggcat gaaatatct                                                    19

<210>    22
<211>    19

<212>	RNA
<213>	Artificial Sequence

<220>
<223>	Sense sequence of siRNA 1 with dTdT attached to the 3' end


<400>	22
guaaagaggc acuagcaaa	19


<210>	23
<211>	19
<212>	RNA
<213>	Artificial Sequence

<220>
<223>	Antisense sequence of siRNA 1 with dTdT attached to the 3' end


<400>	23
uuugcuagug ccucuuuac	19


<210>	24
<211>	19
<212>	RNA
<213>	Artificial Sequence

<220>
<223>	Sense sequence of siRNA 2 with dTdT attached to the 3' end


<400>	24
gcacuagcaa aguccgaga	19


<210>	25
<211>	19
<212>	RNA
<213>	Artificial Sequence

<220>
<223>	Antisense sequence of siRNAs 2 and 21 with dTdT attached to the
	3' end


<400>	25
ucucggacuu ugcuagugc	19

<210>	26
<211>	19
<212>	RNA
<213>	Artificial Sequence

<220>
<223>	Sense sequence of siRNA 3 with dTdT attached to the 3' end

<400>	26
cagcaaagcc aauuuauca	19

<210>	27
<211>	19
<212>	RNA
<213>	Artificial Sequence

<220>
<223>	Antisense sequence of siRNA 3 with dTdT attached to the 3' end

<400>	27
ugauaaauug gcuuugcug	19

<210>	28
<211>	19
<212>	RNA
<213>	Artificial Sequence

<220>
<223>	Sense sequence of siRNA 4 with dTdT attached to the 3' end

<400>	28
cuaugaugau caacucauu	19

<210>	29
<211>	19
<212>	RNA
<213>	Artificial Sequence

<220>
<223>	Antisense sequence of siRNA 4 with dTdT attached to the 3' end

<400>	29
aaugaguuga ucaucauag	19

<210>	30
<211>	19
<212>	RNA
<213>	Artificial Sequence

<220>
<223>	Sense sequence of siRNA 5 with dTdT attached to the 3' end


<400>	30
caaucauacu gcugacaua	19


<210>	31
<211>	19
<212>	RNA
<213>	Artificial Sequence

<220>
<223>	Antisense sequence of siRNA 5 with dTdT attached to the 3' end


<400>	31
uaugucagca guaugauug	19


<210>	32
<211>	19
<212>	RNA
<213>	Artificial Sequence

<220>
<223>	Sense sequence of siRNA 6 with dTdT attached to the 3' end


<400>	32
cucuagaugc ucagacuuu	19


<210>	33
<211>	19
<212>	RNA
<213>	Artificial Sequence

<220>
<223>	Antisense sequence of siRNA 6 with dTdT attached to the 3' end


<400>	33
aaagucugag caucuagag	19

<210>	34
<211>	19
<212>	RNA
<213>	Artificial Sequence

<220>
<223>	Sense sequence of siRNA 7 with dTdT attached to the 3' end


<400>	34
ucuggauugc auuccuaca	19


<210>	35
<211>	19
<212>	RNA
<213>	Artificial Sequence

<220>
<223>	Antisense sequence of siRNA 7 with dTdT attached to the 3' end


<400>	35
uguaggaaug caauccaga	19


<210>	36
<211>	19
<212>	RNA
<213>	Artificial Sequence

<220>
<223>	Sense sequence of siRNA 8 with dTdT attached to the 3' end


<400>	36
cuggauugca uuccuacau	19


<210>	37
<211>	19
<212>	RNA
<213>	Artificial Sequence

<220>
<223>	Antisense sequence of siRNA 8 with dTdT attached to the 3' end


<400>	37

auguaggaau gcaauccag

<210> 38
<211> 19
<212> RNA
<213> Artificial Sequence

<220>
<223> Sense sequence of siRNA 9 with dTdT attached to the 3' end

<400> 38
gcacaaagca agccagauu 19

<210> 39
<211> 19
<212> RNA
<213> Artificial Sequence

<220>
<223> Antisense sequence of siRNA 9 with dTdT attached to the 3' end

<400> 39
aaucuggcuu gcuuugugc 19

<210> 40
<211> 19
<212> RNA
<213> Artificial Sequence

<220>
<223> Sense sequence of siRNA 10 with dTdT attached to the 3' end

<400> 40
cugcuuuaau aggacacuu 19

<210> 41
<211> 19
<212> RNA
<213> Artificial Sequence

<220>
<223> Antisense sequence of siRNA 10 with dTdT attached to the 3' end

<400>    41
aaguguccua uuaaagcag                                                    19

<210>    42
<211>    19
<212>    RNA
<213>    Artificial Sequence

<220>
<223>    Sense sequence of siRNA 11 with dTdT attached to the 3' end

<400>    42
cagguugugg uuucucgau                                                    19

<210>    43
<211>    19
<212>    RNA
<213>    Artificial Sequence

<220>
<223>    Antisense sequence of siRNA 11 with dTdT attached to the 3' end

<400>    43
aucgagaaac cacaaccug                                                    19

<210>    44
<211>    19
<212>    RNA
<213>    Artificial Sequence

<220>
<223>    Sense sequence of siRNA 12 with dTdT attached to the 3' end

<400>    44
cugguuauca cugggaaga                                                    19

<210>    45
<211>    19
<212>    RNA
<213>    Artificial Sequence

<220>
<223>    Antisense sequence of siRNA 12 with dTdT attached to the 3' end

<400>    45
ucuucccagu gauaaccag                                                    19

<210>    46
<211>    19
<212>    RNA
<213>    Artificial Sequence

<220>
<223>    Sense sequence of siRNA 13 with dTdT attached to the 3' end

<400>    46
uugguccugc caugaauaa                                                    19

<210>    47
<211>    19
<212>    RNA
<213>    Artificial Sequence

<220>
<223>    Antisense sequence of siRNA 13 with dTdT attached to the 3' end

<400>    47
uuauucaugg caggaccaa                                                    19

<210>    48
<211>    19
<212>    RNA
<213>    Artificial Sequence

<220>
<223>    Sense sequence of siRNA 14 with dTdT attached to the 3' end

<400>    48
agacaagcau cuucaguua                                                    19

<210>    49
<211>    19
<212>    RNA
<213>    Artificial Sequence

<220>
<223>    Antisense sequence of siRNA 14 with dTdT attached to the 3' end

<400>    49
uaacugaaga ugcuugucu                                                    19

<210>    50
<211>    19
<212>    RNA
<213>    Artificial Sequence

<220>
<223>    Sense sequence of siRNA 15 with dTdT attached to the 3' end

<400>    50
ucgcucuaau ucagagaua                                                    19

<210>    51
<211>    19
<212>    RNA
<213>    Artificial Sequence

<220>
<223>    Antisense sequence of siRNA 15 with dTdT attached to the 3' end

<400>    51
uaucucugaa uuagagcga                                                    19

<210>    52
<211>    19
<212>    RNA
<213>    Artificial Sequence

<220>
<223>    Sense sequence of siRNA 16 with dTdT attached to the 3' end

<400>    52
ucagagauaa ucuguugua                                                    19

<210>    53
<211>    19
<212>    RNA
<213>    Artificial Sequence

<220>

<223>    Antisense sequence of siRNA 16 with dTdT attached to the 3' end

<400>    53
uacaacagau uaucucuga                                                    19

<210>    54
<211>    19
<212>    RNA
<213>    Artificial Sequence

<220>
<223>    Sense sequence of siRNA 17 with dTdT attached to the 3' end

<400>    54
gugagaauau acacuuaca                                                    19

<210>    55
<211>    19
<212>    RNA
<213>    Artificial Sequence

<220>
<223>    Antisense sequence of siRNAs 17 and 22 with dTdT attached to the
         3' end

<400>    55
uguaagugua uauucucac                                                    19

<210>    56
<211>    19
<212>    RNA
<213>    Artificial Sequence

<220>
<223>    Sense sequence of siRNA 18 with dTdT attached to the 3' end

<400>    56
gguguugucu caauaucaa                                                    19

<210>    57
<211>    19
<212>    RNA
<213>    Artificial Sequence

<220>
<223>    Antisense sequence of siRNA 18 with dTdT attached to the 3' end

<400>    57
uugauauuga gacaacacc                                                    19

<210>    58
<211>    19
<212>    RNA
<213>    Artificial Sequence

<220>
<223>    Sense sequence of siRNA 19 with dTdT attached to the 3' end

<400>    58
cauuuggaua ggcuuguaa                                                    19

<210>    59
<211>    19
<212>    RNA
<213>    Artificial Sequence

<220>
<223>    Antisense sequence of siRNA 19 with dTdT attached to the 3' end

<400>    59
uuacaagccu auccaaaug                                                    19

<210>    60
<211>    19
<212>    RNA
<213>    Artificial Sequence

<220>
<223>    Sense sequence of siRNA 20 with dTdT attached to the 3' end

<400>    60
ccaaaggcau gaaauaucu                                                    19

<210>    61
<211>    19
<212>    RNA

<213>    Artificial Sequence

<220>
<223>    Antisense sequence of siRNAs 20 and 23 with dTdT attached to the
         3' end

<400>    61
agauauuuca ugccuuugg                                                    19

<210>    62
<211>    16
<212>    RNA
<213>    Artificial Sequence

<220>
<223>    Sense sequence of siRNA 21

<400>    62
cuagcaaagu ccgaga                                                       16

<210>    63
<211>    16
<212>    RNA
<213>    Artificial Sequence

<220>
<223>    Sense sequence of siRNA 22

<400>    63
agaauauaca cuuaca                                                       16

<210>    64
<211>    16
<212>    RNA
<213>    Artificial Sequence

<220>
<223>    Sense sequence of siRNA 23

<400>    64
gauugcauuc cuacau                                                       16

<210>    65

<211>    19
<212>    RNA
<213>    Artificial Sequence

<220>
<223>    Sense sequence of siRNA 24, having phosphorothioate bonded dTdT
         attached to the 3' end

<400>    65
gcacuagcaa aguccgaga                                                    19

<210>    66
<211>    19
<212>    RNA
<213>    Artificial Sequence

<220>
<223>    Antisense sequence of siRNA 24,having phosphorothioate bonded
         dTdT attached to the 3' end, and 2'-OH of the ribose rings within
         the 1st and 2nd nucleic acids are substituted with 2'-O-Me

<400>    66
ucucggacuu ugcuagugc                                                    19

<210>    67
<211>    19
<212>    RNA
<213>    Artificial Sequence

<220>
<223>    Sense sequence of siRNA 25, having phosphorothioate bonded dTdT
         attached to the 3' end, and 2'-OH of the ribose rings within the
         1st and 2nd nucleic acids are substituted with 2'-O-Me

<400>    67
gcacuagcaa aguccgaga                                                    19

<210>    68
<211>    19
<212>    RNA
<213>    Artificial Sequence

<220>
<223>    Antisense sequence of siRNA 25, having phosphorothioate bonded
         dTdT attached to the 3' end, and 2'-OH of the ribose rings within

the 1st and 2nd nucleic acids are substituted with 2'-O-Me

<400> 68
ucucggacuu ugcuagugc 19

<210> 69
<211> 19
<212> RNA
<213> Artificial Sequence

<220>
<223> Sense sequence of siRNA 26, having phosphorothioate bonded dTdT
attached to the 3' end, and 2'-OH of the ribose rings within the
1st, 2nd nucleic acids, and the nucleic acids having U base are
substituted with 2'-O-Me

<400> 69
gcacuagcaa aguccgaga 19

<210> 70
<211> 19
<212> RNA
<213> Artificial Sequence

<220>
<223> Antisense sequence of siRNA 26, having phosphorothioate bonded
dTdT attached to the 3' end, and 2'-OH of the ribose rings within
the 1st and 2nd nucleic acids are substituted with 2'-O-Me

<400> 70
ucucggacuu ugcuagugc 19

<210> 71
<211> 19
<212> RNA
<213> Artificial Sequence

<220>
<223> Sense sequence of siRNA 27, having phosphorothioate bonded dTdT
attached to the 3' end, and 2'-OH of the ribose rings within the
nucleic acids having G base are substituted with 2'-O-Me, and the
nucleic acids having U base are with 2'-F

<400> 71

gcacuagcaa aguccgaga 19

<210> 72
<211> 19
<212> RNA
<213> Artificial Sequence

<220>
<223> Antisense sequence of siRNA27, phosphorothioate bonded dTdT to
the 3'end, and 2'-OH of the riboserings in the 1st, 2nd, and the
nucleic acids having G base are substituted with 2'-O-Me, and
having U base are with 2'-F, but the 10, 11th nucleic acids are
not

<400> 72
ucucggacuu ugcuagugc 19

<210> 73
<211> 19
<212> RNA
<213> Artificial Sequence

<220>
<223> Sense sequence of siRNA 28, having phosphorothioate bonded dTdT
attached to the 3' end, and 2'-OH of the ribose rings within the
1st and 2nd nucleic acids are substituted with 2'-O-Me, and ENA
is inserted in the nucleic acid of the 5' end

<400> 73
gcacuagcaa aguccgaga 19

<210> 74
<211> 19
<212> RNA
<213> Artificial Sequence

<220>
<223> Antisense sequence of siRNA 28, having phosphorothioate bonded
dTdT attached to the 3' end, and 2'-OH of the ribose rings within
the 1st and 2nd nucleic acids are substituted with 2'-O-Me

<400> 74
ucucggacuu ugcuagugc 19

<210>	75
<211>	19
<212>	RNA
<213>	Artificial Sequence

<220>
<223>	Sense sequence of siRNA 29, having phosphorothioate bonded dTdT
	attached to the 3' end

<400>	75
gugagaauau acacuuaca	19

<210>	76
<211>	19
<212>	RNA
<213>	Artificial Sequence

<220>
<223>	Antisense sequence of siRNA 29, having phosphorothioate bonded
	dTdT attached to the 3' end, and 2'-OH of the ribose rings within
	the 1st and 2nd nucleic acids are substituted with 2'-O-Me

<400>	76
uguaagugua uauucucac	19

<210>	77
<211>	19
<212>	RNA
<213>	Artificial Sequence

<220>
<223>	Sense sequence of siRNA 30, having phosphorothioate bonded dTdT
	attached to the 3' end, and 2'-OH of the ribose rings within the
	1st and 2nd nucleic acids are substituted with 2'-O-Me

<400>	77
gugagaauau acacuuaca	19

<210>	78
<211>	19
<212>	RNA
<213>	Artificial Sequence

<220>
<223>	Antisense sequence of siRNA 30, having phosphorothioate bonded

dTdT attached to the 3' end, and 2'-OH of the ribose rings within
the 1st and 2nd nucleic acids are substituted with 2'-O-Me

<400>    78
uguaagugua uauucucac                                                    19

<210>    79
<211>    19
<212>    RNA
<213>    Artificial Sequence

<220>
<223>    Sense sequence of siRNA 31, having phosphorothioate bonded dTdT
         attached to the 3' end, and 2'-OH of the ribose rings within the
         1st, 2nd nucleic acids, and the nucleic acids having U base are
         substituted with 2'-O-Me

<400>    79
gugagaauau acacuuaca                                                    19

<210>    80
<211>    19
<212>    RNA
<213>    Artificial Sequence

<220>
<223>    Antisense sequence of siRNA 31, having phosphorothioate bonded
         dTdT attached to the 3' end, and 2'-OH of the ribose rings within
         the 1st and 2nd nucleic acids are substituted with 2'-O-Me

<400>    80
uguaagugua uauucucac                                                    19

<210>    81
<211>    19
<212>    RNA
<213>    Artificial Sequence

<220>
<223>    Sense sequence of siRNA 32, having phosphorothioate bonded dTdT
         attached to the 3' end, and 2'-OH of the ribose rings within the
         nucleic acids having G base are substituted with 2'-O-Me, and the
         nucleic acids having U base are with 2'-F

<400>    81
gugagaauau acacuuaca                                                    19

<210>    82
<211>    19
<212>    RNA
<213>    Artificial Sequence

<220>
<223>    Antisense sequence of siRNA32, phosphorothioate bonded dTdT to
         the 3'end, and 2'-OH of the riboserings in the 1st, 2nd, and the
         nucleic acids having G base are substituted with 2'-O-Me, and
         having U base are with 2'-F, but the 10, 11th nucleic acids are
         not

<400>    82
uguaagugua uauucucac                                                    19

<210>    83
<211>    19
<212>    RNA
<213>    Artificial Sequence

<220>
<223>    Sense sequence of siRNA 33, having phosphorothioate bonded dTdT
         attached to the 3' end, and 2'-OH of the ribose rings within the
         1st and 2nd nucleic acids are substituted with 2'-O-Me, and ENA
         is inserted in the nucleic acid of the 5' end

<400>    83
gugagaauau acacuuaca                                                    19

<210>    84
<211>    19
<212>    RNA
<213>    Artificial Sequence

<220>
<223>    Antisense sequence of siRNA 33, having phosphorothioate bonded
         dTdT attached to the 3' end, and 2'-OH of the ribose rings within
         the 1st and 2nd nucleic acids are substituted with 2'-O-Me

<400>    84
uguaagugua uauucucac                                                    19

<210>	85
<211>	19
<212>	RNA
<213>	Artificial Sequence

<220>
<223>	Sense sequence of siRNA 34, having phosphorothioate bonded dTdT
	attached to the 3' end


<400>	85
gugagaauau acacuuaca	19


<210>	86
<211>	19
<212>	RNA
<213>	Artificial Sequence

<220>
<223>	Antisense sequence of siRNA 34, having phosphorothioate bonded
	dTdT attached to the 3' end, and 2'-OH of the ribose ring within
	the 2nd nucleic acid are substituted with 2'-O-Me


<400>	86
uguaagugua uauucucac	19


<210>	87
<211>	19
<212>	RNA
<213>	Artificial Sequence

<220>
<223>	Sense sequence of siRNA 35, having phosphorothioate bonded dTdT
	attached to the 3' end


<400>	87
ccaaaggcau gaaauaucu	19


<210>	88
<211>	19
<212>	RNA
<213>	Artificial Sequence

<220>
<223>	Antisense sequence of siRNA 35, having phosphorothioate bonded

dTdT attached to the 3' end, and 2'-OH of the ribose rings within
the 1st and 2nd nucleic acids are substituted with 2'-O-Me

<400> 88
agauauuuca ugccuuugg 19

<210> 89
<211> 19
<212> RNA
<213> Artificial Sequence

<220>
<223> Sense sequence of siRNA 36, having phosphorothioate bonded dTdT
attached to the 3' end, and 2'-OH of the ribose rings within the
1st and 2nd nucleic acids are substituted with 2'-O-Me

<400> 89
ccaaaggcau gaaauaucu 19

<210> 90
<211> 19
<212> RNA
<213> Artificial Sequence

<220>
<223> Antisense sequence of siRNA 36, having phosphorothioate bonded
dTdT attached to the 3' end, and 2'-OH of the ribose rings within
the 1st and 2nd nucleic acids are substituted with 2'-O-Me

<400> 90
agauauuuca ugccuuugg 19

<210> 91
<211> 19
<212> RNA
<213> Artificial Sequence

<220>
<223> Sense sequence of siRNA 37, having phosphorothioate bonded dTdT
attached to the 3' end, and 2'-OH of the ribose rings within the
1st, 2nd nucleic acids, and the nucleic acids having U base are
substituted with 2'-O-Me

<400> 91

ccaaaggcau gaaauaucu 19

<210> 92
<211> 19
<212> RNA
<213> Artificial Sequence

<220>
<223> Antisense.sequence of siRNA 37, having phosphorothioate bonded
dTdT attached to the 3' end, and 2'-OH of the ribose rings within
the 1st and 2nd nucleic acids are substituted with 2'-O-Me

<400> 92
agauauuuca ugccuuugg 19

<210> 93
<211> 19
<212> RNA
<213> Artificial Sequence

<220>
<223> Sense sequence of siRNA 38, having phosphorothioate bonded dTdT
attached to the 3' end, and 2'-OH of the ribose rings within the
nucleic acids having G base are substituted with 2'-O-Me, and the
nucleic acids having U base are with 2'-F

<400> 93
ccaaaggcau gaaauaucu 19

<210> 94
<211> 19
<212> RNA
<213> Artificial Sequence

<220>
<223> Antisense sequence of siRNA38, phosphorothioate bonded dTdT to
the 3'end, and 2'-OH of the riboserings in the 1st, 2nd, and the
nucleic acids having G base are substituted with 2'-O-Me, and
having U base are with 2'-F, but the 10, 11th nucleic acids are
not

<400> 94
agauauuuca ugccuuugg 19

<210>	95
<211>	19
<212>	RNA
<213>	Artificial Sequence

<220>
<223>	Sense sequence of siRNA 39, having phosphorothioate bonded dTdT
	attached to the 3' end, and 2'-OH of the ribose rings within the
	1st and 2nd nucleic acids are substituted with 2'-O-Me, and ENA
	is inserted in the nucleic acid of the 5' end

<400>	95
ccaaaggcau gaaauaucu	19

<210>	96
<211>	19
<212>	RNA
<213>	Artificial Sequence

<220>
<223>	Antisense sequence of siRNA 39, having phosphorothioate bonded
	dTdT attached to the 3' end, and 2'-OH of the ribose rings within
	the 1st and 2nd nucleic acids are substituted with 2'-O-Me

<400>	96
agauauuuca ugccuuugg	19

<210>	97
<211>	19
<212>	RNA
<213>	Artificial Sequence

<220>
<223>	Sense sequence of siRNA 40, having phosphorothioate bonded dTdT
	attached to the 3' end

<400>	97
ccaaaggcau gaaauaucu	19

<210>	98
<211>	19
<212>	RNA
<213>	Artificial Sequence

<220>

<223>    Antisense sequence of siRNA 40, having phosphorothioate bonded
         dTdT attached to the 3' end, and 2'-OH of the ribose ring within
         the 2nd nucleic acid are substituted with 2'-O-Me

<400>    98
agauauuuca ugccuuugg                                                    19

<210>    99
<211>    21
<212>    RNA
<213>    Artificial Sequence

<220>
<223>    Sense sequence of APOB-1 siRNA

<400>    99
gucaucacac ugaauaccaa u                                                 21

<210>    100
<211>    23
<212>    RNA
<213>    Artificial Sequence

<220>
<223>    Antisense sequence of APOB-1 siRNA

<400>    100
auugguauuc agugugauga cac                                               23